# EUROPÄISCHE PATENTANMELDUNG

(11) **EP 3 133 189 A1**
(43) Veröffentlichungstag der Anmeldung: **22.02.2017**
(21) Anmeldenummer: 16182787.8
(22) Anmeldetag: 04.08.2016
(51) Int. Cl.: C25B 3/10, C07C 43/23

(54) **VERFAHREN ZUR HERSTELLUNG VON UNSYMMETRISCHEN OCO-PINCERLIGANDEN AUS DER GRUPPE DER M-TERPHENYLVERBINDUNGEN**

(30) Priorität: 21.08.2015 DE 102015216001
(71) Anmelder: Evonik Degussa GmbH, 45128 Essen (DE)
(72) Erfinder: Dyballa, Katrin Marie, 45657 Recklinghausen (DE); Franke, Robert, 45772 Marl (DE); Fridag, Dirk, 45721 Haltern am See (DE); Waldvogel, Siegfried R., 55435 Gau-Algesheim (DE); Elsler, Bernd, 53127 Bonn (DE); Wiebe, Anton, 56567 Neuwied (DE); Lips, Sebastian, 55595 Hüffelsheim (DE)

(57) **Zusammenfassung**

Die vorliegende Erfindung betrifft ein Verfahren zur Herstellung unsymmetrischer Verbindungen aus der Gruppe der m-Terphenyle, die als unsymmetrische OCO-Pincerliganden verwendet werden können, umfassend die Verfahrensschritte a) Umsetzen eines ersten substituierten oder unsubstituierten Phenols mit einem 1,3-disubstituierten Aren, welches in 2 und 5 Stellung ebenfalls substituiert sein kann, unter Erhalt eines Phenol-Aren-Kupplungsprodukts, b) optional Schützen der OH-Gruppe des Phenol-Aren-Kupplungsprodukts mit einer Schutzgruppe unter Erhalt eines geschützten Phenol-Aren-Kupplungsprodukts und c) Umsetzen des Phenol-Aren-Kupplungsprodukts aus a) oder b) mit einem zweiten substituierten oder unsubstituierten Phenol unter Erhalt eines unsymmetrischen m-Terphenyls, mit der Maßgabe, dass das erste Phenol und das zweite Phenol unterschiedlich substituiert sind, welches dadurch gekennzeichnet ist, dass mindestens einer der Verfahrensschritte a) oder c) als elektrochemischer Verfahrensschritt durchgeführt wird.

## Beschreibung

Die vorliegende Erfindung betrifft ein Verfahren zur Herstellung unsymmetrischer Verbindungen aus der Gruppe der m-Terphenyle (nachfolgend gegebenenfalls auch m-Terphenylverbindungen oder unsymmetrische OCO-Pincerliganden genannt) umfassend die Verfahrensschritte a) Umsetzen eines ersten substituierten oder unsubstituierten Phenols mit einem 1,3-disubstituierten Aren, welches in 2 und 5 Stellung ebenfalls substituiert sein kann, unter Erhalt eines Phenol-Aren-Kupplungsprodukts, b) optional Schützen der OH-Gruppe des Phenol-Aren-Kupplungsprodukts mit einer Schutzgruppe unter Erhalt eines geschützten Phenol-Aren-Kupplungsprodukts und c) Umsetzen des Phenol-Aren-Kupplungsprodukts aus a) oder b) mit einem zweiten substituierten oder unsubstituierten Phenol unter Erhalt eines unsymmetrischen m-Terphenyls, mit der Maßgabe, dass das erste Phenol und das zweite Phenol unterschiedlich substituiert sind, welches dadurch gekennzeichnet ist, dass mindestens einer der Verfahrensschritte a) oder c) als elektrochemischer Verfahrensschritt durchgeführt wird.

Als m-Terphenylen werden üblicherweise 1,3-Diphenylbenzole bezeichnet. Verbindungen aus der Gruppe der m-Terphenyle enthalten folglich eine 1,3-Diphenylbenzolleitstruktur. Im Rahmen der vorliegenden Erfindung versteht man unter unsymmetrischen OCO-Pincerliganden Verbindungen, die eine 1,3-Diphenylbenzoleinheit aufweisen, wobei die beiden äußeren Ringe keine Arene, sondern Phenole sind. Die OH-Gruppen der beiden Phenolbausteine sind jeweils ortho zu der Arenkomponente substituiert.

Unter unsymmetrischen OCO-Pincerliganden versteht man im Rahmen der vorliegenden Erfindung Verbindungen, bei denen sich die beiden Phenole strukturell unterscheiden, also verschieden sind. Im Rahmen der Anmeldung muss die Arenkomponenten, die zwischen den beiden verschiedenen Phenolkomponenten angeordnet ist, nicht zwingend symmetrisch sein, d.h. es ist nicht notwendig, dass man, wenn man eine Spiegelebene in das Arenmolekül legt, die eine Seite auf die andere Seite abbildbar ist.

Weiterhin wird im Rahmen der vorliegenden Erfindung folgende Nummerierung der Stellungen in der Arenkomponente (B) vorgenommen:

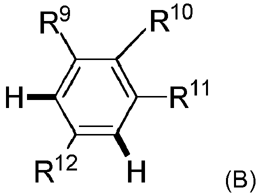

R⁹ ist definiert als Substituent am C-Atom 1 des Benzolringes, R¹⁰ als Substituent am C-Atom 2, R¹¹ als Substituent am C-Atom 3, R¹² als Substituent am C-Atom 5.

Im Rahmen der vorliegenden Erfindung werden unter substituierten Phenolen solche verstanden, bei denen die Wasserstoffatome an einem oder mehreren Ringkohlenstoffatomen substituiert wurden.

Die klassische Synthese von OCO-Pincerliganden ist bisher auf symmetrische Derivate beschränkt. Die bekannten Verfahren ermöglichen die C_{Ar}-C_{Ar}-Bindungsknüpfung beispielsweise durch Ullmann-Kupplung (B. G. Pring, Acta Chem. Scand. 1973, 27, 3873-3880), Grignard-Wurtz-Kupplung (R. S. Grewal, H. Hart, T. K. Vinod, J. Org. Chem. 1992, 57, 2721-2726.), oder Palladium-katalysierten Kupplungsreaktionen (S. Sarkar, A. R. Carlson, M. K. Veige, J. M. Falkowski, K. A. Abboud, A. S. Veige, J. Am. Chem. Soc. 2008, 130, 1116-1117). Vor den eigentlichen Kupplungsreaktionen ist der Schutz der Hydroxyfunktion notwendig, welche in den meisten Fällen mittels Methylierung erreicht wird. In allen bekannten Fällen ist die Durchführung der Reaktion unter Feuchtigkeitsausschluss und anaeroben Bedingungen notwendig.

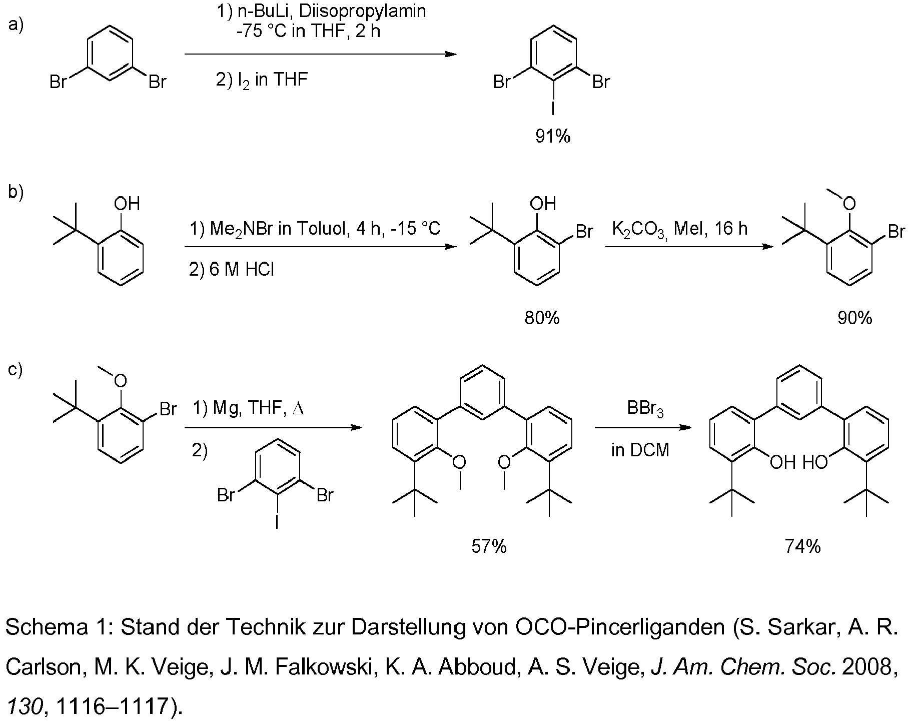

Ein großer Nachteil der bekannten Methoden zur Kreuzkupplung von Phenolderivaten mit Arenen ist die Notwendigkeit trockener Lösungsmittel und eines Luftausschlusses während der Kupplungsreaktion. Bei klassischen Kreuzkupplungsmethoden ist der Schutz der Hydroxyfunktionalität in den meisten Fällen notwendig. Weiterhin werden für eine regioselektive Kreuzkupplung Abgangsfunktionalitäten in den eingesetzten Substraten eingeführt, die nach der eigentlichen Kupplungsreaktion als umweltproblematische Reaktionsabfälle anfallen (siehe Schema 1). Die Toleranz funktioneller Gruppen wird durch die eingesetzten Reagenzien oft eingeschränkt, so dass vor allem wenig substituierte Derivate eingesetzt werden können. Während der Reaktion treten oft toxische Nebenprodukte auf (wie beispielsweise Bromverbindungen), die vom gewünschten Produkt aufwendig abgetrennt und teuer entsorgt werden müssen. Ferner muss die Reaktion über sehr viele Stufen hergestellt werden, was ebenfalls sehr unvorteilhaft ist. Knapper werdende Rohstoffe machen die Darstellung von Ligandensystemen durch Palladium katalysierte Kreuzkupplungen oder chemische Oxidationsmittel sehr kostspielig.

Die Darstellung unsymmetrischer Pincerliganden ist bisher klassisch nicht möglich. Auch elektrochemische Verfahren zur Kreuzkupplung von Phenolen zu unsymmetrischen m-Terphenylverbindungen sind bisher nicht bekannt.

Aufgabe der vorliegenden Erfindung war deshalb die Bereitstellung eines einfachen Verfahrens zur Herstellung von unsymmetrischen OCO-Pincerliganden aus der Gruppe der m-Terphenylverbindungen bereitzustellen, welches einen oder mehrere Nachteile des Standes der Technik vermeidet.

Überraschenderweise wurde gefunden, dass diese Aufgabe dadurch gelöst werden kann, dass in einem Verfahren, welches zwei Verfahrensschritte der Umsetzung aufweist, mindestens einer der Verfahrensschritte als elektrochemischer Verfahrensschritt durchgeführt wird.

Gegenstand der vorliegenden Erfindung ist deshalb ein Verfahren zur Herstellung unsymmetrischer Verbindungen aus der Gruppe der m-Terphenyle, so genannte unsymmetrische OCO-Pincerliganden, umfassend die Verfahrensschritte
a) Umsetzen eines ersten substituierten oder unsubstituierten Phenols mit einem 1,3-disubstituierten Aren, welches in 2 und 5 Stellung ebenfalls substituiert sein kann, unter Erhalt eines Phenol-Aren-Kupplungsprodukts und
b) optional Schützen der OH-Gruppe des Phenol-Aren-Kupplungsprodukts mit einer Schutzgruppe unter Erhalt eines geschützten Phenol-Aren-Kupplungsprodukts
c) Umsetzen des Phenol-Aren-Kupplungsprodukts aus a) oder b) mit einem zweiten substituierten oder unsubstituierten Phenol unter Erhalt eines unsymmetrischen m-Terphenyls,
mit der Maßgabe, dass das erste Phenol und das zweite Phenol unterschiedlich substituiert sind,
dadurch gekennzeichnet,
dass mindestens einer der Verfahrensschritte a) oder c) als elektrochemischer Verfahrensschritt durchgeführt wird.

Ebenfalls Gegenstand der vorliegenden Erfindung sind Verbindungen der Formel (CDC') 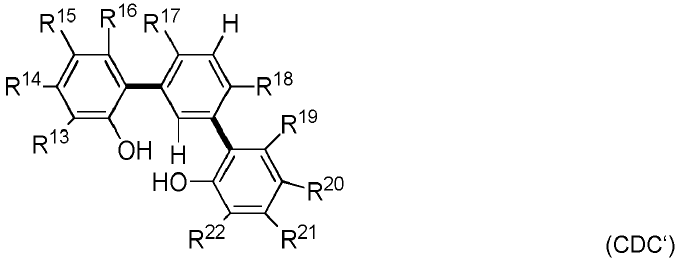 wie nachfolgend definiert.

Außerdem ist Gegenstand der vorliegenden Erfindung die Verwendung der erfindungsgemäßen Verbindungen als Ligand zur Herstellung von Metall-Ligand-Katalysatorsystemen.

Das erfindungsgemäße Verfahren hat den Vorteil, dass durch die teilweise oder vollständige Umsetzung mittels elektrochemischer Verfahrensschritte die Herstellung von unsymmetrische m-Terphenylverbindungen wesentlich vereinfacht wird, da ein Feuchtigkeitsausschluss oder die Einhaltung von anaerobe Reaktionsbedingungen nicht gewährleistet werden muss. Dies führt unter anderem zu einem geringeren Aufwand bei der Durchführung des Verfahrens.

Die Durchführung eines Verfahrensschritt oder mehrerer Verfahrensschritte als elektrochemische Verfahrensschritte hat außerdem den Vorteil, dass die Bildung von unerwünschten Nebenprodukten deutlich reduziert werden kann.

Durch die elektrochemische Kreuzkupplung entfällt der Einsatz teurer und giftiger Katalysatoren, so wie die Einführung aktivierender Abgangsfunktionalitäten vor der Phenol-Aren-Kupplungsreaktion (erfindungsgemäßer Verfahrensschritt a)).

Je nach Ausführungsart des erfindungsgemäßen Verfahrens erlaubt das Verfahren erstmals die direkte Synthese unsymmetrischer Verbindungen bei geringem synthetischem Aufwand und gleichzeitig hoher Toleranz funktioneller Gruppen in den eingesetzten Substraten.

Des Weiteren kann bei dem erfindungsgemäßen Verfahren teilweise oder vorzugsweise vollständig auf den Einsatz von chemischen Oxidationsmitteln verzichtet werden. Auf diese Weise wird die Gefahr der Bildung von Nebenprodukten bzw. der Verunreinigung der Verbindungen durch Rückstände der Oxidationsmittel vermieden.

Das erfindungsgemäße Verfahren hat außerdem den Vorteil, dass eine große Vielfalt unterschiedlicher m-Terphenylverbindungen dargestellt werden können, die nach den aus dem Stand der Technik bekannten Verfahren nicht oder nur unter großem Aufwand zugänglich waren.

Insbesondere hat das erfindungsgemäße Verfahren den Vorteil, dass mit ihm die erfindungsgemäßen m-Terphenylverbindungen (m-Terphenyle) hergestellt werden können.

Das erfindungsgemäße Verfahren, die erfindungsgemäßen Verbindungen sowie deren Verwendung werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt, insbesondere bezüglich der in Bezug genommenen Sachverhalte vollständig zum Offenbarungsgehalt der vorliegenden Erfindung gehören. Werden nachfolgend Angaben in Prozent gemacht, so handelt es sich, wenn nicht anders angegeben um Angaben in Vol-%. Werden nachfolgend Mittelwerte angegeben, so handelt es sich, wenn nicht anders angegeben, um das Zahlenmittel. Werden nachfolgend Stoffeigenschaften, wie z. B. Viskositäten oder ähnliches angegeben, so handelt es sich, wenn nicht anders angegeben, um die Stoffeigenschaften bei 25 °C.

Das erfindungsgemäße Verfahren zur Herstellung unsymmetrischer Verbindungen aus der Gruppe der m-Terphenyle umfassend die Verfahrensschritte a) Umsetzen eines ersten substituierten oder unsustituierten Phenols mit einem 1,3-disubstituierten Aren, welches in 2 und 5 Stellung ebenfalls substituiert sein kann, unter Erhalt eines Phenol-Aren-Kupplungsprodukts, b) optional Schützen der OH-Gruppe des Phenol-Aren-Kupplungsprodukts mit einer Schutzgruppe unter Erhalt eines geschützten Phenol-Aren-Kupplungsprodukts und c) Umsetzen des Phenol-Aren-Kupplungsprodukts aus a) oder b) mit einem zweiten substituierten oder unsubstituierten Phenol unter Erhalt eines unsymmetrischen m-Terphenyls, mit der Maßgabe, dass das erste Phenol und das zweite Phenol unterschiedlich substituiert sind, zeichnet sich dadurch aus, dass mindestens einer der Verfahrensschritte a) oder c), vorzugsweise Verfahrensschritt c), bevorzugt beide Verfahrensschritte a) und c) als elektrochemischer Verfahrensschritt durchgeführt wird.

Vorzugsweise werden in dem erfindungsgemäßen Verfahren sowohl als erstes Phenol als auch als zweites Phenol, substituierte Phenole eingesetzt.

Es kann vorteilhaft sein, das Phenol-Aren-Kupplungsprodukt aus dem Schritt a) zu isolieren, bevor es dem Verfahrensschritt c) zugeführt wird.

Es ist erfindungsgemäß bevorzugt, in den Verfahrensschritten a), b) und c) nicht umgesetzte Edukte zurückzugewinnen, was vorzugsweise destillativ erfolgt. Die Zurückgewonnen Edukte können den entsprechenden Verfahrensschritten a), b) bzw. c) wieder als Einsatzstoff zugeführt werden.

Vorzugsweise wird der elektrochemische Verfahrensschritt/werden die elektrochemischen Verfahrensschritte so durchgeführt, dass
aa) eine Mischung mindestens eines Lösemittels und mindestens eines Leitsalzes erzeugt wird,
bb) zu dieser Mischung die umzusetzenden Verbindungen gegeben werden, wobei das substituierte Phenol (erstes oder zweites substituiertes Phenol) im molaren Unterschuss bezogen auf die andere umzusetzende Komponente (disubstituiertes Aren bzw. Phenol-Aren Kupplungsprodukt) zugegeben wird,
cc) mindestens zwei Elektroden in die unter bb) erhaltene Reaktionslösung eingebracht und eine Spannung an die Elektroden angelegt wird,
dd) die Komponenten zu dem entsprechenden Kupplungsprodukt (Phenol-Aren- oder m-Terphenyl-Kupplungsprodukt) umgesetzt werden,
ee) Abschalten der Spannung, sowie gegebenenfalls,
ff) Isolierung und/oder Aufreinigung des Umsetzungsproduktes.

Werden beide Verfahrensschritte a) und c) als elektrochemische Verfahrensschritte durchgeführt, so wird das erfindungsgemäße Verfahren vorzugsweise so durchgeführt, dass es die Verfahrensschritte:
a1) Mischen mindestens eines Lösemittels und eines Leitsalzes,
a2) Zugabe eines ersten substituierten oder unsubstituierten Phenols und eines 1,3-disubstituierten Arens, welches in 2 und 5 Stellung ebenfalls substituiert sein kann, in die Mischung aus a1), wobei das 1,3-disubstituierten Aren in einem molaren Überschuss bezogen auf das erste substituierte oder unsubstituierten Phenol zugegeben wird,
a3) Einbringen von mindestens zwei Elektroden in die Reaktionslösung und Anlegen einer Spannung an die Elektroden
a4) Kupplung des ersten substituierten oder unsubstituierten Phenols an das 1,3-disubstituierten Aren zu einem Phenol-Aren-Kupplungsprodukt,
a5) Abschalten der Spannung, Abtrennen und ggf. Aufreinigung des Phenol-Aren-Kupplungsproduktes,
b1) optional Schützen der OH-Gruppe des Phenol-Aren-Kupplungsprodukts mit einer Schutzgruppe unter Erhalt eines geschützten Phenol-Aren-Kupplungsprodukts und
c1) Mischen des Phenol-Aren-Kupplungsproduktes aus Schritt a5) oder des geschützten Phenol-Aren-Kupplungsprodukts aus Schritt b1) mit einer Reaktionslösung aus mindestens einem Lösemittel und einem darin enthaltenen Leitsalz,
c2) Zugabe eines zweiten substituierten oder unsubstituierten Phenols, das unterschiedlich zum ersten substituierten oder unsubstituierten Phenol substituiert ist, wobei das Phenol-Aren-Kupplungsprodukt in einem molaren Überschuss bezogen auf das zweite substituierte oder unsubstituierten Phenol in der Reaktionslösung vorliegt,
c3) Einbringen von mindestens zwei Elektroden in die Reaktionslösung und Anlegen einer Spannung an die Elektroden,
c4) Kupplung des zweiten substituierten oder unsubstituierten Phenols an das Phenol-Aren-Kupplungsprodukt zu einem unsymmetrischen m-Terphenyl,
c5) Abschalten der Spannung und ggf.
c6) Isolierung und/oder Aufarbeitung des unsymmetrischen m-Terphenyls.

Die elektrochemischen Verfahrensschritte können z. B. in Anlehnung an die von a.) A. Kirste, B. Elsler, G. Schnakenburg, S. R. Waldvogel, J. Am. Chem. Soc. 2012, 134, 3571-3576, b.) A. Kirste, S. Hayashi, G. Schnakenburg, I. M. Malkowsky, F. Stecker, A. Fischer, T. Fuchigami, S. R. Waldvogel, Chem. Eur. J. 2011, 17, 14164-14169, c.) B. Elsler, D. Schollmeyer, K. M. Dyballa, R. Franke, S. R. Waldvogel, Angew. Chem. Int. Ed. 2014, 53, 5210-5213, und d.) A. Kirste, M. Nieger, I. M. Malkowsky, F. Stecker, A. Fischer, S. R. Waldvogel, Chem. Eur. J. 2009, 15, 2273-2277 beschriebenen Verfahren zur elektrochemischen Kupplung von Kohlenstoff-Kohlenstoff-Bindungen, durchgeführt werden.

Die elektochemischen Verfahrenschritte können in allen geeigneten, aus dem Stand der Technik bekannten Elektrolysezellen durchgeführt werden. Bevorzugt wird in dem erfindungsgemäßen Verfahren eine Flanschzelle, eine Becherglaszelle oder eine Screeningzelle eingesetzt. Solche Zellen sind literaturbeschrieben und die ersten beiden Glaszellen können, z.B. unter dieser Bezeichnung bei HWS Labortechnik Mainz bezogen werden.

Die Zelle weist mindestens zwei Elektroden auf. Als Elektroden können handelsübliche Elektroden eingesetzt werden. Als Anoden können vorzugsweise z. B. BDD (0,015 mm bor-dotierter Diamant auf Silizium oder 0,05 mm bor-dotiertem Diamant auf Niob), Platin-, isostatische Graphit- oder Glaskohlenstoffelektroden eingesetzt werden. Als Kathoden werden vorzugsweise BDD-, Nickelnetz- oder Glaskohlenstoffelektroden eingesetzt. Solche BDD-Elektroden (bor-dotierter Diamant auf einem Träger, wie z.B. Niob oder Silizium) sind z. B. unter der Bezeichnung DIACHEM bei der Firma Condias GmbH Itzehoe erhältlich. Die restlichen Elektroden sind von gängigen Chemikalien- und Materiallieferanten, wie z.B. Goodfellow oder Aldrich erhältlich.

Der elektrochemische Verfahrensschritt oder die elektrochemischen Verfahrensschritte werden vorzugsweise in Gegenwart eines Lösemittels durchgeführt. Als Lösemittel wird vorzugsweise ein Lösemittel aus der Gruppe von Acetonitril, Propylencarbonat, Methylcarbonat, Nitromethan, Ethylenglycoldimethylether, Methansulfonsäure, Benzol, Toluol, Wasser, Methanol, Ethanol, Propanol, Isopropanol, halogenierte Lösemittel oder halogenierte oder nicht halogenierte Säuren oder Mischungen dieser eingesetzt.

Bevorzugt werden als Lösemittel eine Carbonsäure, vorzugsweise Ameisensäure, zugegeben, eine fluorierte Carbonsäure oder ein fluorierter Alkohol, vorzugsweise Trifluoressigsäure oder 1,1,1,3,3,3-Hexafluor-2-propanol (HFIP), bevorzugt 1,1,1,3,3,3-Hexafluor-2-propanol eingesetzt.

Besonders bevorzugt wird als Lösemittel Methanol, Ameisensäure Trifluoressigsäure, Hexafluorisopropanol oder Mischungen dieser, vorzugsweise Methanol, Hexafluorisopropanol oder Mischungen dieser, und besonders bevorzugt Hexafluorisopropanol (1,1,1,3,3,3-Hexafluor-2-propanol) verwendet.

Der elektrochemische Verfahrensschritt oder die elektrochemischen Verfahrensschritte werden vorzugsweise in Gegenwart mindestens eines Leitsalzes durchgeführt wird, wobei als Leitsalze bevorzugt solche eingesetzte werden, die ausgewählt sind aus der Gruppe von Tetra(C₁-C₆-alkyl)-ammonium- oder 1,3-Di(C₁-C₆-alkyl)imidazoliumsalzen, mit der Maßgabe, dass die Alkylgruppen Halogen-substituiert, insbesondere mit Fluor-substituiert sein können. Vorzugsweise werden solche Leitsalze eingesetzt, die Gegenionen aufweisen, die ausgewählt sind aus der Gruppe umfassend Arsenat, Sulfat, Hydrogensulfat, Alkylsulfat, Alkylphosphat, Perchlorat, Fluorid, Arylsulfat, Hexafluorphosphat und Tetrafluorborat. Bevorzugte Leitsalze sind solche aus der Gruppe von quaternären Ammoniumborate, Ammoniumfluoralkylphosphate, Ammoniumfluoralkylarsenate, Ammoniumtrifluor-methylsulfonate, Ammoniumbis(fluoromethansulfon)imide, Ammoniumtris- (fluoromethansulfonyl)methide, Methyltributylammoniummethylsulfat, Methyltriethylammoniummethylsulfat, Tetrabutyl-ammoniumhexafluorophosphat, Tetraethylammoniumtetrafluoroborat, Lithiumhexafluorophosphat oder Tetraethylammoniumtetrafluoroborat. Besonders bevorzugt wird Methyltriethylammoniummethylsulfat oder Bu₃NMe⁺MeOSO₃⁻ (Methyltributylammoniummethylsulfat), ganz besonders bevorzugt Methyltributylammoniummethylsulfat als Leitsalz eingesetzt.

Das Leitsalz wird vorzugsweise in einer Konzentration von 0,001 bis 10 mol/L, bevorzugt 0,01 bis 1 mol/L, besonders bevorzugt von 0,075 bis 0,125 mol/L und ganz besonders bevorzugt von 0,09 mol/L bezogen auf die Reaktionsmischung eingesetzt.

Vorzugsweise wird in dem erfindungsgemäßen Verfahren jeweils so viel an erstem bzw. zweitem substituierten Phenol eingesetzt, dass die Konzentration von 0,001 bis 5 mol/L, vorzugsweise von 0,05 bis 0,5 mol/L, bevorzugt von 0,1 bis 0,3 mol/L und besonders bevorzugt 0,15 mol/L beträgt.

Der elektrochemische Verfahrensschritt oder die elektrochemischen Verfahrensschritte werden vorzugsweise bei Raumtemperatur oder erhöhter Temperatur durchgeführt. Bevorzugt werden der elektrochemische Verfahrensschritt oder die elektrochemischen Verfahrensschritte bei einer Temperatur in der Elektrolysezelle im Bereich von 25 bis 80 °C, bevorzugt von 35 bis 70 °C und bevorzugt von 45 bis 55 °C durchgeführt.

Der elektrochemische Verfahrensschritt oder die elektrochemischen Verfahrensschritte werden vorzugsweise galvanostatisch durchgeführt.

Bei der Durchführung des elektrochemischen Verfahrensschritts oder der elektrochemischen Verfahrensschritte wird die Stromstärke vorzugsweise so gewählt, dass die Stromdichte von 1 bis 10 mA/cm², vorzugsweise von 2 bis 5,5 mA/cm², vorzugsweise von 2,5 bis 3 mA/cm² und bevorzugt 2,8 mA/cm² beträgt. Zur Durchführung des elektrochemischen Verfahrensschritts oder der elektrochemischen Verfahrensschritte wird vorzugsweise eine (Klemm-)Spannung zwischen den Elektroden im Bereich von 2 bis 10 V, bevorzugt 2,5 bis 7,5 V und bevorzugt von 3 bis 6 V angelegt.

Es kann vorteilhaft sein, wenn so viel an 1,3-disubstituiertem Aren und erstem substituierten oder unsubstituierten Phenol eingesetzt wird, dass das molare Verhältnis des 1,3-disubstituierten Arens zum ersten substituierten oder unsubstituierten Phenol im Bereich von 1,5 : 1 bis 6 : 1, bevorzugt im Bereich 2 : 1 bis 3 : 1 liegt.

Das molare Verhältnis des Phenol-Aren Kupplungsproduktes zum zweiten substituierten oder unsubstituierten Phenol liegt vorzugsweise im Bereich von 1,5 : 1 bis 6 : 1, bevorzugt im Bereich von 2 : 1 bis 3 : 1.

Besonders bevorzugt werden die Mengenverhältnisse so gewählt, dass in Schritt a) das molare Verhältnis des 1,3-disubstituierten Arens zum ersten substituierten oder unsubstituierten Phenol im Bereich von 1,5 zu 1 bis 6 : 1, bevorzugt im Bereich 2 : 1 bis 3 : 1 und in Schritt b) das molare Verhältnis des Phenol-Aren Kupplungsproduktes zum zweiten substituierten oder unsubstituierten Phenol im Bereich von 1,5 : 1 bis 6 : 1, bevorzugt im Bereich von 2 : 1 bis 3 : 1 liegt.

In dem erfindungsgemäßen Verfahren werden als erste substituierte oder unsubstituierte Phenole vorzugsweise solche der Formel (A) 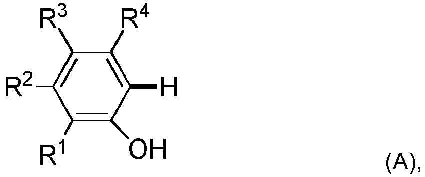 als zweite substituierte oder unsubstituierte Phenole werden vorzugsweise solche der Formel (A'), 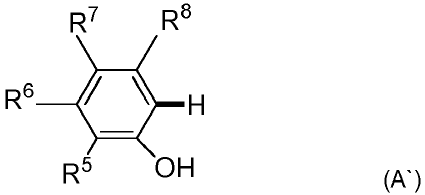 und als 1,3-disubstituierte, die in 2- und 5-Stellung ebenfalls substituiert sein können, Arene werden vorzugsweise solche der Formel (B) 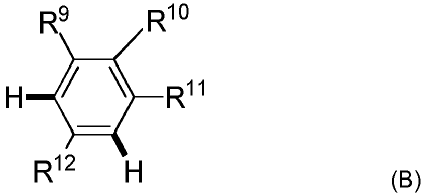 eingesetzt, worin die Reste
und R¹, R², R³, R⁴, R⁵, R⁶, R⁷, R⁸, R¹⁰ und R¹² unabhängig voneinander ausgewählt sind aus der Gruppe bestehend aus Wasserstoff, (C₁-C₁₂)-Alkyl, O-(C₁-C₁₂)-Alkyl, (C₆-C₂₀)-Aryl, O-(C₆-C₂₀)-Aryl und Halogen, vorzugsweise bestehend aus Wasserstoff, (C₁-C₁₂)-Alkyl, O-(C₁-C₁₂)-Alkyl und Halogen, besonders bevorzugt bestehend aus Wasserstoff, (C₁-C₁₂)-Alkyl, O-(C₁-C₁₂)-Alkyl,
worin R⁹ und R¹¹ unabhängig voneinander ausgewählt sind aus der Gruppe bestehende aus (C₁-C₁₂)-Alkyl, O-(C₁-C₁₂)-Alkyl, (C₆-C₂₀)-Aryl, O-(C₆-C₂₀)-Aryl und, Halogen, vorzugsweise bestehend aus (C₁-C₁₂)-Alkyl, O-(C₁-C₁₂)-Alkyl und Halogen, besonders bevorzugt bestehend aus (C₁-C₁₂)-Alkyl, O-(C₁-C₁₂)-Alkyl,
und worin innerhalb der Paare R¹/ R⁵, R²/R⁶, R³/R⁷ oder R⁴/R⁸ mindestens ein Paar aus verschiedenen Resten besteht.

Im Rahmen der vorliegenden Erfindung wird unter Halogen Fluor, Chlor, Brom oder Iod verstanden, insbesondere Fluor, Chlor und Brom. Unter dem Begriff Alkyl wird im Rahmen der vorliegenden Erfindung ein linearer oder verzweigter Kohlenwasserstoffrest verstanden, bei dem gegebenenfalls ein oder mehrere Wasserstoffatome, vorzugsweise durch Halogenatome oder Hydroxyl- oder Alk(yl)oxy-Gruppen, substituiert sein können. Aryl bedeutet im Rahmen der vorliegenden Erfindung aromatische Kohlenwasserstoffreste, wie z. B. Phenyl- (C₆H₅-), Naphthyl- (C₁₀H₇-), Anthryl- (C₁₄H₉-), vorzugsweise Phenyl, bei denen gegebenenfalls ein oder mehrere Wasserstoffatome, vorzugsweise durch Alkyl- oder Alk(yl)oxy-Gruppen, substituiert sein können.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl-,oder Dodecyl-. Die Reste "(C₁-C₁₂)-Alkyl und O-(C₁-C₁₂)-Alkyl" können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen

Der Ausdruck -(C₆-C₂₀)-Aryl umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Substituierte -(C₆-C₂₀)-Arylgruppen können, in Abhängigkeit von der Ringgröße, einen oder mehrere (z.B. 1, 2, 3, 4 oder 5) Substituenten aufweisen. Diese Substituenten sind vorzugsweise unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl,-CO-(C₆-C₂₀)-Aryl, -COOH, -OH, -SO₃H; -SO₃Na, -NO₂, -CN, -NH₂, -N[(C₁-C₁₂)-Alkyl] Nachfolgend wird durch zwei Reaktionsschemen beispielhaft die Durchführung des erfindungsgemäßen Verfahrens aufgezeigt, wobei hierbei beide Verfahrensschritte a) und c) als elektrochemische Verfahrensschritte durchgeführt werden. Die in den Schemen verwendeten Reste R¹ bis R¹² sind wie oben definiert. Die Kupplung eines substituierten oder unsubstituierten Phenols an ein 1,3-disubstituiertes Aren, das in 2- und 5-Stellung ebenfalls substituiert sein kann, im ersten Verfahrensschritt a) (Schema 2) führt zum Phenol-Aren Kupplungsprodukt (AB).

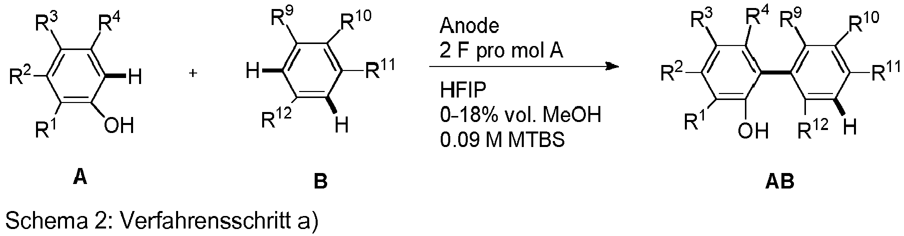

Im zweiten Verfahrensschritt c) wird das Phenol-Aren Kupplungsprodukt (AB) mit einem weiteren substituierten oder unsubstituierten Phenol (A') zur Verbindung ABA' umgesetzt und so in einen unsymmetrischen OCO-Pincerliganden überführt (Schema 3).

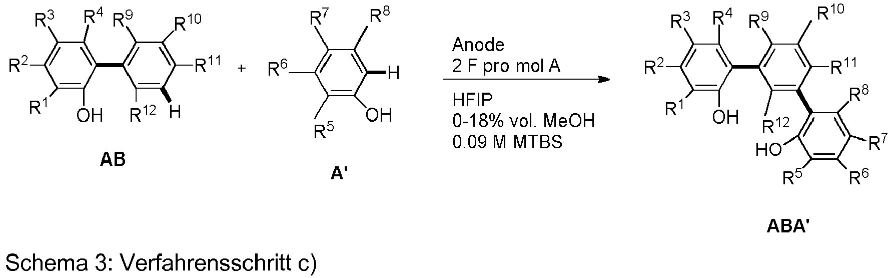

Solche Verbindungen sind auf klassischem Wege bisher nicht beschrieben bzw. nur mit hohem synthetischen Aufwand zugänglich.

Es kann vorteilhaft sein, wenn zwischen Verfahrensschritt a) und Verfahrensschritt c) ein Verfahrensschritt b) durchgeführt wird, bei dem die Hydroxygruppe des Phenol-Aren-Kupplungsprodukts durch eine geeignete Schutzgruppe geschützt wird. Ein solcher Verfahrensschritt ist insbesondere dann vorteilhaft, wenn beide Verfahrensschritte a) und c) als elektrochemische Verfahrensschritte durchgeführt werden.

In einer Variante des erfindungsgemäßen Verfahrens werden deshalb in Verfahrensschritt c) geschützte Phenol-Aren-Kupplungsprodukte, vorzugsweise solche der Formel (A"B) 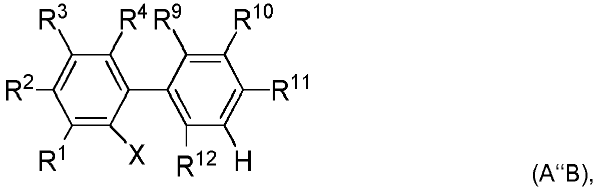 zusammen mit dem zweiten substituierten oder unsubstituierten Phenol, vorzugsweise einem der Formel (A'), 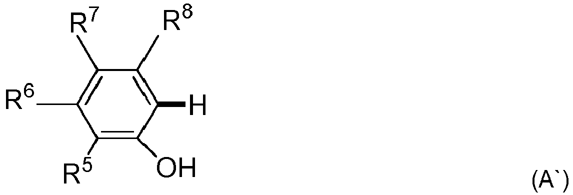 eingesetzt, worin X gleich -OR' ist, mit R' = einer Schutzgruppe, vorzugsweise einer Silyl-Schutzgruppe, bevorzugt einer -trimethylsilyl, -triethylsilyl, -triisopropylsilyl, *-tert.-*butyldiphenylsilyl oder (1,1-Dimethylethyl)-dimethylsilyl-Schutzgruppe, besonders bevorzugt eine -triisopropylsilyl- oder (1,1-Dimethylethyl)-dimethylsilyl-Schutzgruppe, und R¹, R², R³, R⁴, R⁵, R⁶, R⁷, R⁸, R¹⁰ und R¹² unabhängig voneinander ausgewählt sind aus der Gruppe enthaltend Wasserstoff, (C₁-C₁₂)-Alkyl, O-(C₁-C₁₂)-Alkyl, (C₆-C₂₀)-Aryl, O-(C₆-C₂₀)-Aryl und Halogen, vorzugsweise bestehend aus Wasserstoff, (C₁-C₁₂)-Alkyl, O-(C₁-C₁₂)-Alkyl und Halogen, bevorzugt bestehend aus Wasserstoff, (C₁-C₁₂)-Alkyl, O-(C₁-C₁₂)-Alkyl, worin die Reste R⁹ und R¹¹ unabhängig voneinander ausgewählt sind aus der Gruppe enthaltend (C₁-C₁₂)-Alkyl, O-(C₁-C₁₂)-Alkyl, (C₆-C₂₀)-Aryl, O-(C₆-C₂₀)-Aryl und, Halogen, vorzugsweise bestehend aus (C₁-C₁₂)-Alkyl, O-(C₁-C₁₂)-Alkyl und Halogen, bevorzugt bestehend aus (C₁-C₁₂)-Alkyl, O-(C₁-C₁₂)-Alkyl, und
worin innerhalb der Paare R¹/ R⁵, R²/R⁶, R³/R⁷ oder R⁴/R⁸ mindestens ein Paar aus verschiedenen Resten besteht.

Der Verfahrensschritt b) kann so durchgeführt werden, dass das Phenol-Aren-Kupplungsprodukts aus Verfahrensschritt a) vor oder nach einer Aufarbeitung mit einer geeigneten Verbindung umgesetzt wird. Vorzugsweise wird eine Silyl-Schutzgruppe eingefügt. Besonders bevorzugt wird als Verbindung (1,1-Dimethylethyl)-dimethylsilylchlorid und Tri(methylethyl)-silylchlorid eingesetzt.

Silyl-Schutzgruppen und deren Einführung sind dem Fachmann bekannt und kann der gängigen Fachliteratur entnommen werden (siehe hierzu auch: P. G. M. Wuts, T. W. Greene "Greenes's Protective Groups in Organic Synthesis", fourth edition, 2007, John Wiley and Sons; Hoboken, New Jersey)

Überraschenderweise haben die Erfinder gefunden, dass bei Einsatz eines Phenol-Aren-Kupplungsprodukts, welches eine geschützte Hydroxylgruppe, insbesondere eine durch Umsetzung mit Silylgruppe geschützte Hydroxylgruppe aufweist, in Verfahrensschritt c), wenn dieser als elektrochemischer Verfahrensschritt durchgeführt wird, die Schutzgruppe wieder eliminiert wird, so dass eine m-Terphenylverbindung erhalten wird, die wieder zwei OH-Gruppen aufweist.

Überraschenderweise haben die Erfinder außerdem gefunden, dass durch das Einführen einer Schutzgruppe, insbesondere einer Silylgruppe, in das Phenol-Aren-Kupplungsprodukt bei der anschließenden Kupplung im elektrochemisch durchgeführten Verfahrensschritt c) die Ausbeute an m-Terphenylverbindung, die wieder zwei OH-Gruppen aufweist, gesteigert werden kann. Somit kann die Effizienz des Gesamtverfahrens nochmals deutlich verbessert werden.

Das erfindungsgemäße Verfahren wird vorzugsweise so durchgeführt, dass die Umsetzung in den elektrochemischen Verfahrensschritten, bevorzugt die gesamte Umsetzung ohne die Verwendung von organischen Oxidationsmitteln durchgeführt wird.

Mit Hilfe des erfindungsgemäßen Verfahrens kann eine große Vielfalt unterschiedlicher m-Terphenylverbindungen, insbesondere solche der Formel (ABA') 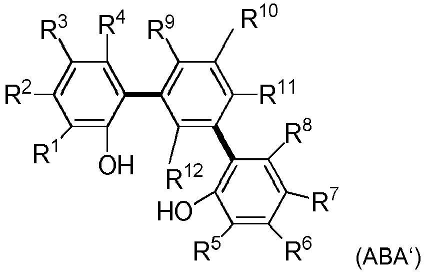 mit X, Y, und R¹ bis R¹² wie oben definiert, hergestellt werden. Mit dem erfindungsgemäßen Verfahren können insbesondere die erfindungsgemäßen Verbindungen der Formel (CDC') hergestellt werden. Die erfindungsgemäßen Verbindungen der Formel (CDC') 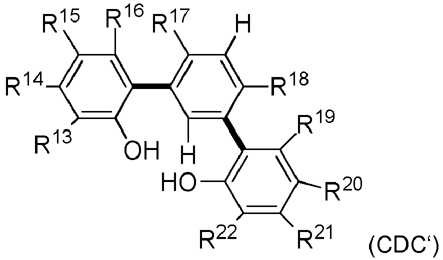
worin die Reste R¹³, R¹⁴, R¹⁵, R¹⁶, R¹⁹, R²⁰, R²¹ und R²² unabhängig voneinander ausgewählt sind aus der Gruppe bestehende aus Wasserstoff, (C₁-C₁₂)-Alkyl, O-(C₁-C₁₂)-Alkyl, (C₆-C₂₀)-Aryl, O-(C₆-C₂₀)-Aryl, Halogen, vorzugsweise bestehend aus Wasserstoff, (C₁-C₁₂)-Alkyl, O-(C₁-C₁₂)-Alkyl, Halogen, besonders bevorzugt bestehend aus Wasserstoff, (C₁-C₁₂)-Alkyl und O-(C₁-C₁₂)-Alkyl,
worin R¹⁷ und R¹⁸ unabhängig voneinander ausgewählt sind aus der Gruppe bestehende aus (C₁-C₁₂)-Alkyl, O-(C₁-C₁₂)-Alkyl, (C₆-C₂₀)-Aryl, O-(C₆-C₂₀)-Aryl und Halogen, vorzugsweise bestehend aus (C₁-C₁₂)-Alkyl, O-(C₁-C₁₂)-Alkyl und Halogen, besonders bevorzugt bestehend aus (C₁-C₁₂)-Alkyl und O-(C₁-C₁₂)-Alkyl, und
worin innerhalb der Paare R¹³/R²², R¹⁴/R²¹, R¹⁵/R²⁰oder R¹⁶/R¹⁹ mindestens ein Paar aus verschiedenen Resten besteht.

Vorzugsweise sind die Reste R¹³, R¹⁴, R¹⁵, R¹⁶, R¹⁹, R²⁰, R²¹ und R²² unabhängig voneinander ausgewählt aus der Gruppe bestehend aus Wasserstoff, (C₁-C₁₂)-Alkyl, O-(C₁-C₁₂)-Alkyl, Halogen,
und die Reste R¹⁷ und R¹⁸ unabhängig voneinander ausgewählt aus der Gruppe bestehend aus (C₁-C₁₂)-Alkyl, O-(C₁-C₁₂)-Alkyl oder Halogen.

Bevorzugt sind die Reste R¹³ R¹⁴, R¹⁵, R¹⁶, R¹⁹, R²⁰, R²¹ und R²² unabhängig voneinander ausgewählt aus der Gruppe bestehend aus Wasserstoff, (C₁-C₁₂)-Alkyl, O-(C₁-C₁₂)-Alkyl, und die Reste R¹⁷ und R¹⁸ unabhängig voneinander ausgewählt aus der Gruppe bestehend aus (C₁-C₁₂)-Alkyl, O-(C₁-C₁₂)-Alkyl.

Ganz besonders bevorzugte erfindungsgemäße Verbindungen sind solche, die den Formeln (1), (2) oder (3) 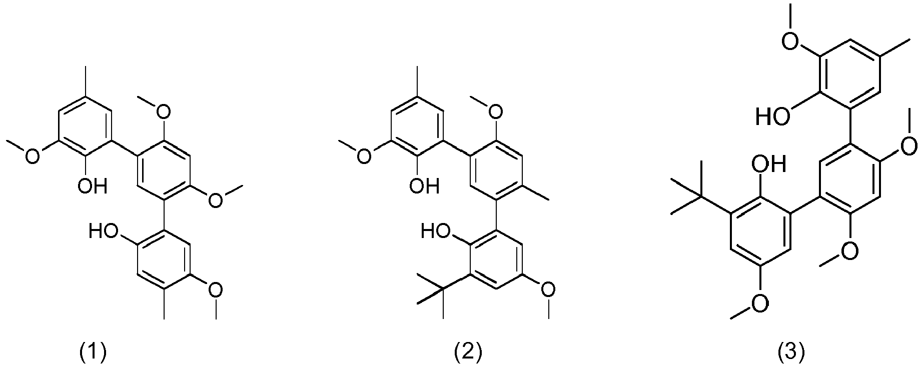 genügen.

Die erfindungsgemäßen Verbindungen können als Ligand zur Herstellung von Metall-Ligand-Katalysatorsystemen verwendet werden.

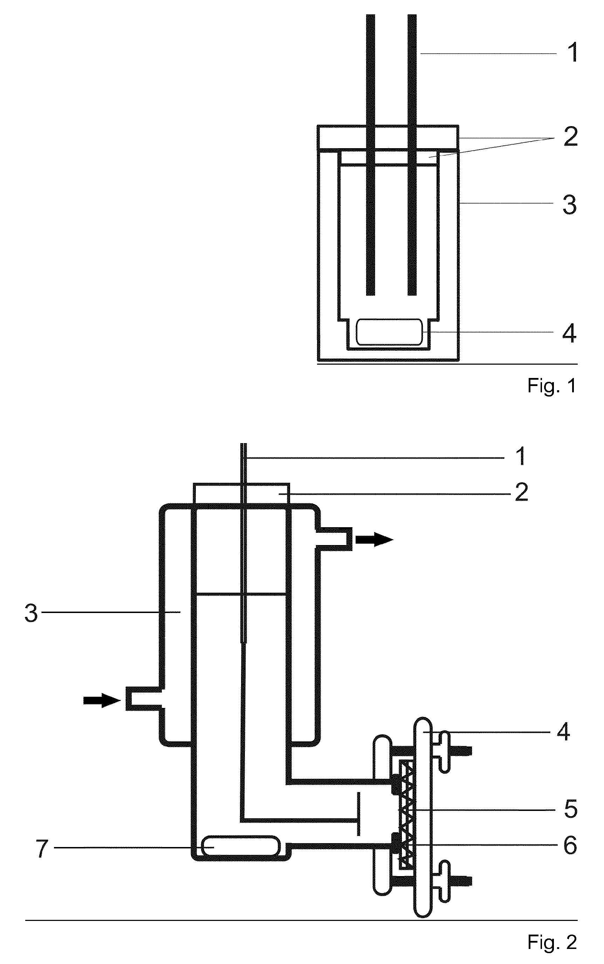

Die vorliegende Erfindung wird an Hand der Figuren 1 bis 4 beispielhaft beschrieben, ohne dass die Erfindung, deren Anwendungsbreite sich aus der gesamten Beschreibung und den Ansprüchen ergibt, auf die in den Figuren abgebildeten Ausführungsformen beschränkt sein soll. In den Figuren 1 bis 4 sind schematische Zeichnungen von Zellen, wie sie im erfindungsgemäßen Verfahren eingesetzt werden können und wie sie insbesondere in den nachfolgenden Beispielen verwendet wurden, abgebildet.
Fig. 1 zeigt schematisch eine Screening-Zelle. In Fig. 2 sind mit 1 die BDD-Elektroden, mit 2 ein Teflon-Deckel, mit 3 ein Teflon-Becher und mit 4 ein Rührmagnet gekennzeichnet.
Fig. 2 zeigt schematisch eine Flanschzelle (Glas). In Fig. 2 sind mit 1 ein Edelstahl-Stab mit befestigter Nickel-Netz-Kathode, mit 2 ein Teflon-Stopfen, mit 3 ein Kühlmantel (Glas), mit 4 eine Schraubzwinge, mit 5 eine BDD-Elektrode, mit 6 eine EPDM Dichtung und mit 7 ein Rührmagnet gekennzeichnet.

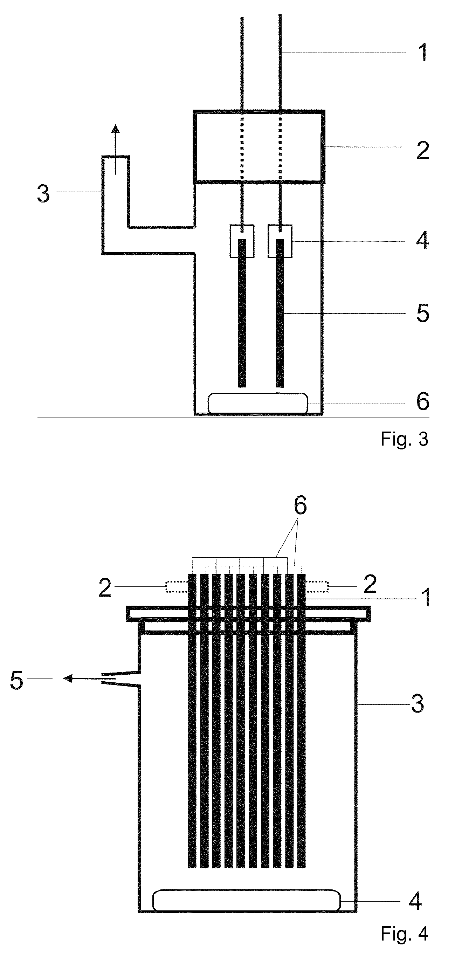

Fig. 3 zeigt schematisch eine Becherglas-Zelle klein (Glas). In Fig. 3 sind mit 1 ein Edelstahl-Stab zur Kontaktierung, mit 2 ein Teflon-Stopfen, mit 3 ein Zellen-Auslass/Anschluss, z. B. für einen Dimroth-Kühler, mit 4 Elektrodenhalterungen (Edelstahl), mit 5 Glaskohlenstoff-Elektroden und mit 6 ein Rührmagnet gekennzeichnet.
Fig. 4 zeigt schematisch eine Becherglaszelle groß (Glas). In Fig. 4 sind mit 1 die BDD-Elektroden, mit 2 die Anschlüsse zur Kontaktierung, mit 3 der Glasmantel, mit 4 ein Rührmagnet, mit 5 ein Zellen-Auslass/Anschluss, z.B. für einen Dimroth-Kühler und mit 6 ein elektrisch leitender Kontakt (Edelstahlfolie) bezeichnet.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Experimenteller Teil

### Allgemeine Methoden

### Chromatographie (GC/GCMS)

Präparative Flüssigchromatographie zur Auftrennung von Stoffgemischen wurde unter Verwendung von Kieselgel 60 M (0,040-0,063 mm) der Firma MACHERY-NAGEL GMBH & CO. KG, Düren bei einem Maximaldruck von 2 bar durchgeführt. Alle verwendeten Eluenten (Essigsäureethylester, technische Qualität; Cyclohexan, technische Qualität) wurden vorab destillativ am Rotationsverdampfer gereinigt.
Dünnschichtchromatographie (DC) wurde an PSC-Fertigplatten Kieselgel 60 F₂₅₄ der Firma MERCK KGaA, Darmstadt durchgeführt. Detektion der verschiedenen Substanzen erfolgte zunächst unter UV-Licht und anschließend durch Anfärben mittels Cer-Molybdatophosphorsäure-Reagenz (5,6 g Molybdatophosphorsäure, 2,2 g Cer(IV)-sulfat-Tetrahydrat und 13,3 g konz. Schwefelsäure auf 200 mL Wasser) und anschließendem Erhitzen durch einen Heißluftfön.

### Gaschromatographie (GC/GCMS)

Die gaschromatographischen Untersuchungen (GC) von Produktgemischen und Reinsubstanzen erfolgte mit Hilfe des Gaschromatographen GC-2010 der Firma *Shimadzu,* Japan. Es wird an einer Quarzkapillarsäule HP-5 der Firma *Agilent Technologies,* USA (Länge: 30 m; Innendurchmesser: 0,25 mm; Filmdicke der kovalent gebundenen stationären Phase: 0,25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min) gemessen. Gaschromatographische Massenspektren (GCMS) von Produktgemischen und Reinsubstanzen wurden mit Hilfe des Gaschromatographen GC-2010 kombiniert mit dem Massendetektor GCMS-QP2010 der Firma *Shimadzu,* Japan aufgenommen. Es wird an einer Quarzkapillarsäule HP-1 der Firma *Agilent Technologies,* USA (Länge: 30 m; Innendurchmesser: 0,25 mm; Filmdicke der kovalent gebundenen stationären Phase: 0,25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min; GCMS: Temperatur der lonenquelle: 200 °C) gemessen.

### Massenspektrometrie

Alle Elektrosprayionisation-Messungen (ESI+) wurden an einem QTof Ultima 3 der Firma *Waters Micromasses,* Milford, Massachusetts durchgeführt. EI-Massenspektren sowie die hochaufgelösten EI-Spektren wurden an einem Gerät des Typs MAT 95 XL Sektorfeldgerät der Firma *Thermo Finnigan,* Bremen, gemessen.

### NMR-Spektroskopie

Die NMR-spektroskopischen Untersuchungen wurden an Multikernresonanzspektrometern des Typs AC 300 oder AV II 400 der Firma *Bruker,* Analytische Messtechnik, Karlsruhe, durchgeführt. Als Lösungsmittel wurde CDCI3 verwendet. Die ¹H- und ¹³C-Spektren wurden gemäß dem Restgehalt an nicht deuteriertem Lösungsmittel nach der *NMR Solvent Data Chart* der Fa. *Cambridge Isotopes Laboratories,* USA, kalibriert. Die Zuordnung der ¹H- und ¹³C-Signale erfolgte teilweise mit Hilfe von H,H-COSY, H,H-NOESY, H,C-HSQC und H,C-HMBC-Spektren. Die chemischen Verschiebungen sind als δ-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten J wurden mit der Anzahl der eingeschlossenen Bindungen in Hertz (Hz) angegeben. Die bei der Signalzuordnung angegebene Nummerierung entspricht der in den Formelschemata angegebenen Bezifferung, die nicht zwingend mit der IUPAC-Nomenklatur übereinstimmen muss.

### Einkristallstrukturanalysen

Die Einkristallstrukturanalysen wurden im Institut für Organische Chemie der Johannes Gutenberg-Universität Mainz an einem Gerät des Typs IPDS 2T der Firma *STOE & Cie GmbH,* Darmstadt durchgeführt.

### Schmelzpunkte

Die relevanten Schmelzpunkte wurden mit Hilfe des Schmelzpunktbestimmungsgerätes SG 2000 der Firma *HW5,* Mainz gemessen und wurden unkorrigiert übernommen.

### Allgemeine Arbeitsvorschriften

### AAV1: Elektrochemische Kreuzkupplung in L-Zellen

5 mmol der zu oxidierenden Spezies A bzw. A' (Unterschusskomponente) wurden mit einem 2-3 fachen Überschuss (10-15 mmol) des Kupplungspartners B bzw. AB in 33 mL 1,1,1,3,3,3-Hexafluorisopropanol (HFIP) bzw. 33 mL (HFIP mit 18 Vol% Methanol (MeOH) bezogen auf die Summe aus HFIP und MeOH) in einer ungeteilten Flanschzelle mit BDD-Anode und Nickelnetzkathode umgesetzt. Als Leitsalz verwendete man Bu₃NMe⁺MeOSO₃⁻ (MTBS) mit einer Konzentration von 0.09 M. Die Elektrolyse erfolgte galvanostatisch. Der Außenmantel der Elektrolysezelle temperierte man über einen Thermostaten auf etwa 10 °C, während die Reaktionsmischung gerührt und mit Hilfe eines Ölbades auf 50 °C erhitzt wurde. Nach Ende der Elektrolyse wurde der Zellinhalt in einen 50 mL Rundhalskolben überführt und das Lösungsmittel unter vermindertem Druck am Rotationsverdampfer bei 50 °C, 200 - 70 mbar entfernt. Mineralisationsprodukte sowie das enthaltene Leitsalz trennte man durch Elution mittels Essigsäureethylester (300 mL) über 50 g Kieselgel 60 ab. Nicht umgesetztes Edukt wurde mittels Kurzwegdestillation an der Kugelrohrdestille zurückerhalten (100 °C, 10⁻³ mbar). Die entstandenen Reaktionsprodukte wurden wie jeweils angegeben säulenchromatographisch getrennt.

**Elektrodenmaterial:**

| | |
|---|---|
| Anode: | BDD (15 µm Diamantschicht) auf Siliciumträger |
| Kathode: | Nickelnetz |

**Elektrolysebedingungen:**

| | |
|---|---|
| Temperatur: | 50 °C |
| Stromdichte: | 2,8 mA/cm² |
| Ladungsmenge: | 2 - 4 F bezogen auf die Unterschusskomponente |
| Klemmspannung: | 3-6V |

### AAV2: Elektrochemische Kreuzkupplung in einer Becherglaszelle mit gestapelter Elektrodenanordnung

120 mmol der zu oxidierenden Spezies A (Unterschusskomponente) wurden mit einem 2 - 3 fachen Überschuss (240 - 360 mmol) des Kupplungspartners B in 750 mL 1,1,1,3,3,3-Hexafluorisopropanol bzw. 750 mL (HFIP mit 18vol% MeOH, bezogen auf die Summe aus HFIP und MeOH) in einer Becherglaszelle mit einer gestapelten Anordnung von 10 BDD-Elektroden (5 mal Anode, 5 mal Kathode) umgesetzt. Als Leitsalz verwendete man MTBS mit einer Konzentration von 0,09 M. Die Elektrolyse erfolgte galvanostatisch. Verdampfendes HFIP wurde mit Hilfe eines Dimrothkühlers redestilliert und der Elektrolyse zugeführt, während die Reaktionsmischung gerührt und mit Hilfe eines Ölbades auf 50 °C erhitzt wurde. Nach Ende der Elektrolyse überführte man den Zellinhalt in einen 1 L Rundhalskolben und entfernte das Lösemittel unter vermindertem Druck am Rotationsverdampfer bei 50 °C, 200-70 mbar. Mineralisationsprodukte sowie das enthaltene Leitsalz wurden durch Elution mittels Essigsäureethylester (2 L) über 300 g Kieselgel 60 abgetrennt. Nicht umgesetztes Edukt wurde mittels Kurzwegdestillation an der Kugelrohrdestille zurückerhalten (100 °C, 10⁻³ mbar).

**Elektrodenmaterial:**

| | |
|---|---|
| Anode: | 5 mal BDD auf Niob |
| Kathode: | 5 mal BDD auf Niob |

**Elektrolysebedingungen:**

| | |
|---|---|
| Temperatur: | 50 °C |
| Stromdichte: | 2,8 mA/cm² |
| Ladungsmenge: | 2 - 4 F bezogen auf die Unterschusskomponente |
| Klemmspannung: | 3-6V |

### AAV3: Elektrochemische Kreuzkupplung in einer Becherglaszelle

3,8 mmol der zu oxidierenden Spezies A (Unterschusskomponente) wurden mit einem 2 - 3 fachen Überschuss (7,6 - 11 mmol) des Kupplungspartners B in 25 mL 1,1,1,3,3,3-Hexafluorisopropanol bzw. 25 mL (HFIP mit 18vol% MeOH, bezogen auf die Summe aus HFIP und MeOH) in einer Becherglaszelle an Glaskohlenstoff Elektroden umgesetzt. Als Leitsalz verwendete man MTBS mit einer Konzentration von 0,09 M. Die Elektrolyse erfolgte galvanostatisch. Verdampfendes HFIP wurde mit Hilfe eines Dimrothkühlers redestilliert und der Elektrolyse zugeführt, während die Reaktionsmischung gerührt und mit Hilfe eines Ölbades auf 50 °C erhitzt wurde. Nach Ende der Elektrolyse überführte man den Zellinhalt in einen 100 mL Rundhalskolben und entfernte das Lösungsmittel unter vermindertem Druck am Rotationsverdampfer bei 50 °C, 200-70 mbar. Mineralisationsprodukte sowie das enthaltene Leitsalz wurden durch Elution mittels Essigsäureethylester (300 mL) über 50 g Kieselgel 60 abgetrennt. Nicht umgesetztes Edukt wurde mittels Kurzwegdestillation an der Kugelrohrdestille zurückerhalten (100 °C, 10⁻³ mbar).

**Elektrodenmaterial:**

| | |
|---|---|
| Anode: | Glaskohlenstoff |
| Kathode: | Glaskohlenstoff |

**Elektrolysebedingungen:**

| | |
|---|---|
| Temperatur: | 50 °C |
| Stromdichte: | 2,8 mA/cm² |
| Ladungsmenge: | 2 - 4 F bezogen auf die Unterschusskomponente |
| Klemmspannung: | 3-6V |

### AAV4: Elektrochemische Kreuzkupplung in einer Screening-Zelle

0,76 mmol der zu oxidierenden Spezies A (Unterschusskomponente) wurden mit einem 2 - 3 fachen Überschuss (1,52 - 2,28 mmol) des Kupplungspartners B in 5 mL 1,1,1,3,3,3-Hexafluorisopropanol bzw. 5 mL (HFIP mit 18 vol% MeOH bezogen auf die Summe aus HFIP und MeOH) in einer Becherglaszelle an Glaskohlenstoff Elektroden umgesetzt. Als Leitsalz verwendete man MTBS mit einer Konzentration von 0,09 M. Die Elektrolyse erfolgte galvanostatisch. Die Reaktionsmischung wurde in einem Edelstahlblock platziert, mit Hilfe eines Heizrührers gerührt und auf 50 °C gehalten. Nach Ende der Elektrolyse überführte man den Zellinhalt in einen 50 mL Rundhalskolben und entfernte das Lösungsmittel unter vermindertem Druck am Rotationsverdampfer bei 50 °C, 200-70 mbar. Mineralisationsprodukte sowie das enthaltene Leitsalz wurden durch Elution mittels Essigsäureethylester (150 mL) über 20 g Kieselgel 60 abgetrennt. Nicht umgesetztes Edukt wurde mittels Kurzwegdestillation an der Kugelrohrdestille zurückerhalten (100 °C, 10⁻³ mbar).

**Elektrodenmaterial:**

| | |
|---|---|
| Anode: | BDD (50 µm) auf Niob |
| Kathode: | BDD (50 µm) auf Niob |

**Elektrolysebedingungen:**

| | |
|---|---|
| Temperatur: | 50 °C |
| Stromdichte: | 2,8 mA/cm² |
| Ladungsmenge: | 2 - 4 F bezogen auf die Unterschusskomponente |
| Klemmspannung: | 3-6V |

### Zweistufige Pincerligandensynthese

### Beispiel 1:

### 3,4'-Dimethoxy-2',5-dimethyl-2-hydroxybiphenyl und 2',3-Dimethoxy-4',5-dimethyl-2-hydroxybiphenyl:

Die Elektrolyse wurde gemäß AAV1 durchgeführt, mit dem Unterschied, dass 1,45 g (10,5 mmol, 2,0 Äquiv.) 4-Methylguajakol und 639 mg (5,23 mmol, 1,0 Äquiv.) 3-Methylanisol eingesetzt wurden. Die Stromdichte betrug 2.8 mA/cm², die Ladungsmenge 4 F pro 3-Methylanisol (Q = 2018 C). Nach Abtrennung des Lösungsmittels wurden die Produktmischung säulenchromatographisch an Kieselgel 60 mit einem Eluenten von 9:1 Volumenverhältnis (CH (Cyclohexan):EE (Ethylacetat)) aufgereinigt. Die Produkte wurden als gelbliche, ölartige Substanzen erhalten.

### 3,4'-Dimethoxy-2',5-dimethyl-2-hydroxybiphenyl

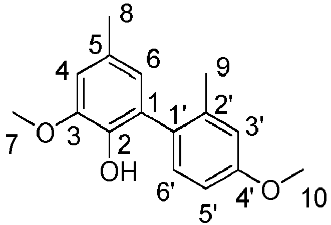

Ausbeute: 206mg (0,8mmol, 15%)
GC (Methode *hart,* HP-5): t_{R} = 13,45 min
R_{f} (Cy:EE = 4:1) = 0,44
¹H-NMR (400 MHz, CDCl₃) δ [ppm] = 2.20 (s, 3H, H-9), 2.32 (2, 3H, H-8), 3.83 (s, 3H, H-10), 3.92 (s, 3H, H-7), 5.44 (bs, 1 H, H-11), 6.57 (m, 1 H, H-4, ⁴J_{4,6} = 1.8 Hz), 6.70 (dd, 1 H, H-6, ⁴J_{4,6} = 1.8 Hz), 6.78-6.82 (dd, 1 H, H-5', ⁴J_{3',5'} = 2.7 Hz, ⁴J_{2',3'} = 8.3 Hz), 6.84 (d, 1 H, H-3', ⁴J_{3',5'} = 2.7 Hz), 7.15 (d, 1 H, H-6', ⁴J_{2',3'} = 8.3 Hz).
¹³C-NMR (101 MHz, CDCl₃) δ [ppm] = 21.14 (C-8), 21.57 (C-9), 55.86 (C-10), 55.95 (C-7), 110.73 (C-5'), 111.21 (C-4), 112.17 (C-3'), 121.85 (C-6), 123.53 (C-1), 125.38 (C-6'), 129.09 (C-5), 131.53 (C-1'), 139.08 (C-2'), 140.93 (C-2), 147.25 (C-3), 155.98 (C-4').
HRMS (ESI, pos. mode): m/z für C₁₆H₁₉O₃ [M+H⁺]: berechnet: 259.1334 gefunden: 259.1332

### 2',3-Dimethoxy-4',5-dimethyl-2-hydroxybiphenyl

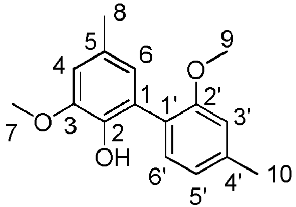

Ausbeute: 225 mg (0,88mmol, 17%)
GC (Methode *hart,* HP-5): t_{R} = 13,32 min
R_{f} (Cy:EE = 4:1) = 0,57
¹H-NMR (400 MHz, CDCl₃) δ [ppm] = 2.33 (s, 3H, H-10), 2.41 (s, 3H, H-8), 3.84 (s, 3H, H-9), 3.91 (s, 3H, H-7), 5.86 (bs, 1 H, H-11), 6.68 (m, 1 H, H-6'), 6.71 (d, 1 H, H-4, ⁴J_{4,6} = 1.7 Hz), 6.84 (m, 1 H, H-3'), 6.86-6.90 (m, 1 H, H-5'), 7.19-7.22 (d, 1 H, H-6', ³J_{5',6'} =7.6 Hz).
¹³C-NMR (101 MHz, CDCl₃) δ [ppm] = 21.14 (C-8), 21.56 (C-10), 55.87 (C-7), 55.95 (C-9), 111.21 (C-1), 112.17 (C-3'), 121.85 (C-4), 123.53 (C-1'), 123.99 (C-5'), 125.38 (C-6'), 129.09 (C-5), 131.53 (C-6), 139.08 (C-4'), 140.86 (C-2), 147.26 (C-3), 155.98 (C-2').
HRMS (ESI, pos. mode): m/z für C₁₆H₁₈O₃Na [M+Na⁺]: berechnet: 281.1154; gefunden: 281.1165

### Beispiel 2:

### 2-Hydroxy-5-methyl-2',3,4'-trimethoxybiphenyl

Die Elektrolyse wurde gemäß AAV2 mit 16,58 g (120 mmol, 1,0 Äquiv.) 4-Methylguajakol und 49,74 g (360 mmol, 3,0 Äquiv.) 1,3-Dimethoxybenzol durchgeführt. Die Stromdichte betrug 2,8 mA/cm², die Ladungsmenge 2 F pro 4-Methylguajakol (Q = 23156 C). Nach Abtrennung des Lösungsmittels und des Leitsalzes wurde das Produkt durch Kurzwegdestillation an der Kugelrohrdestille als gelbes Öl erhalten (140 °C, 10⁻³ mbar). 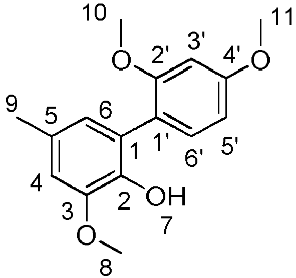
Ausbeute: 9,218 g (33,6 mmol, 28%)
GC (Methode *hart,* HP-5): t_{R} = 14.27 min
R_{f} (Cy:EE=9:1) = 0.42
¹H-NMR (400 MHz, CDCl₃) δ [ppm] = 2.33 (s, 3H, 9-H), 3.83 (s, 3H, 8-H), 3.85 (s, 3H, 10-H), 3.91 (s, 3H, 11-H), 5.76 (bs, 1 H, 7-H), 6.59-6.61 (m, 2H, 5'-H, 6'-H), 6.67 (s, 1 H, 6-H), 6.70 (s, 1 H, 4-H), 7.23 (s, 1 H, 3'-H).
¹³C-NMR (101 MHz, CDCl₃) δ [ppm] = 21.29 (C-7), 55.56 (C-10), 56.02 (C-9), 56.11 (C-8), 99.12, 105.19 (C-5', C-6'), 111.22 (C-4), 119.65 (C-1'), 123.83 (C-6), 125.18 (C-1), 129.14 (C-5), 132.30 (C-3'), 141.04 (C-2), 147.28 (C-3), 157.40 (C-2'), 160.68 (C-4').
HRMS (ESI, pos. mode): m/zfür C₁₆H₁₈O₄ (M+Na⁺): berechnet: 297.1103, gefunden: 297.1096

### Beispiel 3:

### 2,2"-Dihydroxy-5",2'-dimethyl-3-(1,1-dimethylethyl)-3",4',5-trimethoxy[1,1';5',1"]terphenyl

Die Elektrolyse wurde gemäß AAV1 mit 325 mg (1,81 mmol, 1,0 Äquiv.) 2-(1,1-Dimethylethyl)-4-methoxyphenol und 1,40 g (5.42 mmol, 3,0 Äquiv.) 2',3-Dimethoxy-4',5-dimethyl-2-hydroxybiphenyl durchgeführt. Die Stromdichte betrug 2.8 mA/cm², die Ladungsmenge 2 F pro 2-(1,1-Dimethylethyl)-4-methoxyphenol (Q = 350 C). Nach Abtrennung des Lösungsmittels wurde das Reaktionsprodukt säulenchromatographisch an Kieselgel 60 mit einem Laufmittelgemisch 9:1 (CH:EE) durchgeführt. Das Produkt wurde als gelbes Öl erhalten. 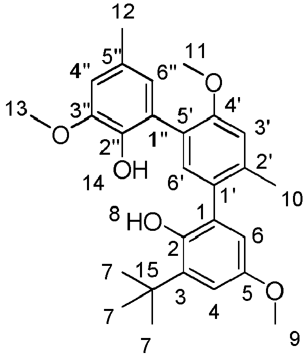
Ausbeute: 126 mg (0,29 mmol, 8%)
GC (Methode *hart,* HP-5): t_{R} = 22,69 min
R_{f} (Cy:EE= 9:1) = 0,51
¹H-NMR (400 MHz, CDCl₃) δ [ppm] = 1.45 (s, 9H, 7-H), 2.30 (s, 3H, 12-H), 2.31 (s, 3H, 10-H), 3.75 (s, 3H, 13-H), 3.91 (s, 3H, 11-H), 3.92 (s, 3H, 9-H), 5.58, 6.06 (bs, 2H, 8-H/14-H), 6.61 (d, 1 H, 6"-H, ⁴J_{6",4"} = 1.2 Hz), 6.69 (d, 1 H, 6-H, ⁴J_{6,4} = 4 Hz), 6.71 (d, 1 H, 4"-H, ⁴J_{4",6"} = 1.2 Hz), 9.91 (d, 1 H, 4-H, ⁴J_{4,6} = 4 Hz), 6.95 (s, 1 H, 3'-H), 7.22 (s, 1 H, 6'-H).
¹³C-NMR (101 MHz, CDCl₃) δ [ppm] = 20.38 (C-12), 21.24 (C-10), 29.84 (C-7), 35.30 (C-15), 55.82 (C-13), 56.15 (C-9), 56.35 (C-11), 110.75 (C-4"), 112.80 (C-6), 113.14 (C-3'), 113.98 (C-4), 123.70 (C-6"), 124.90 (C-1'), 126.98 (C-1), 127.36 (C-1 "), 129.07 (C-5"), 131.60 (C-5'), 134.27 (C-6'), 138.41 (C-2'), 139.03 (C-3), 140.64 (C-2"), 146.35 (C-5), 146.64 (C-3"), 152.88 (C-2), 154.85 (C-4').
HRMS (ESI, pos. mode): *m*/*z* für C₂₇H₃₂O₅ (M+Na⁺): berechnet: 459.2147; gefunden: 459.2147

### Beispiel 4:

### 2,2"-Dihydroxy-4",5-dimethyl-2',3,4',5"-tetramethoxy[1,1';5',1"]terphenyl

Die Elektrolyse wurde gemäß AAV1 mit 250 mg (1.8 mmol, 1.0 Äquiv.) 4-Methoxy-3-methylphenol und 1.49 g (5.4 mmol, 3.0 Äquiv.) 2-Hydroxy-5-methyl-2',3,4'-trimethoxybiphenyl durchgeführt. Die Stromdichte betrug 2.8 mA/cm², die Ladungsmenge 2 F pro 4-Methoxy-3-methylphenol (Q = 350 C). Erste Aufreinigung erfolgte durch Abtrennung der Edukte an der Kugelrohrdestille (120 °C, 10⁻³ mbar). Das Produkt wurde säulenchromatographisch über Kieselgel 60 mit einem Gradienten 4:1, dann 7:3, dann 2:1 (CH:EE) aufgereinigt. Die Terphenylverbindung wurde als gelblicher Feststoff erhalten. 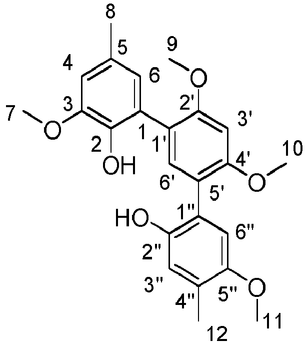
Ausbeute: 126 mg (0,3 mmol, 17%)
GC (Methode *hart,* HP-5): t_{R} = 23,54 min
R_{f} (Cy:EE=2:1)=0,14
¹H-NMR (400 MHz, CDCl₃) δ [ppm] = 2.25 (s, 3H), 2.34 (s, 3H), 3.79 (s, 3H), 3.93 (s, 3H), 3.93 (s, 3H), 3.97 (s, 3H), 5.57 (bs, 2H), 6.76 - 6.70 (m, 4H), 6.87 (s, 1 H), 7.35 (s, 1 H).
¹³C-NMR (101 MHz, CDCl₃) δ [ppm] = 16.06, 21.22, 56.06, 56.09, 56.37, 56.66, 77.36, 96.56, 111.26, 112.87, 119.82, 119.93, 120.96, 123.04, 123.75, 124.46, 127.65, 129.24, 135.01, 141.03, 147.08, 152.09, 156.00, 157.29.
MS (ESI, pos. mode): *m*/*z* für C₂₄H₂₆O₆ (M+Na⁺): berechnet: 433.16
gefunden: 433.16

Anhand der erfindungsgemäßen Beispiele konnte gezeigt werden, dass die unsymmetrischen OCO-Pincerliganden in zufriedenstellenden Ausbeuten nach dem erfindungsgemäßen Verfahren hergestellt werden können.

### Beispiel 5:

### Derivatisierung der Phenol-Aren-Komponente aus Beispiel 2 durch Schutzgruppen Schützung mit (1,1-Dimethylethyl)-dimethylsilylchlorid (TBDMS-chlorid)

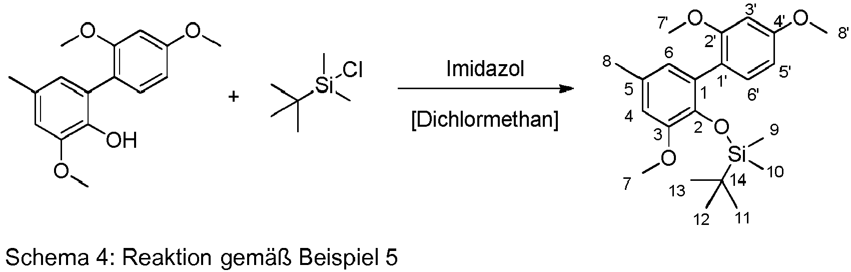

In einem Rundkolben mit Trockenrohr wurden 2,26 g Imidazol (33,2 mmol, 1,1 Äqu.) vorgelegt. 8,29 g (30,2 mmol, 1,0 Äqu.) der AB-Verbindung aus Beispiel 2 (2-Hydroxy-5-methyl-2',3,4'-trimethoxybiphenyl) wurden in ca. 50 mL trockenem Dichlormethan gelöst und in den Kolben überführt. Anschließend wurden 5,92 g (39,3 mmol, 1,3 Äqu.) TBDMS-Chlorid ebenfalls in ca. 50 mL trockenem Dichlormethan gelöst und in Kolben überführt. Weitere 100 mL Dichlormethan wurden hinzugegeben. Der Ansatz wurde dann für 24 h bei Raumtemperatur gerührt. Während der Reaktionszeit entstand ein farbloser, flockiger Niederschlag. Die Isolierung des Produktes erfolgte mittels dreimaliger Extraktion mit je 100 mL Wasser, um das entstandene Salz abzutrennen. Nach Trocknung mittels Natriumsulfat und Entfernung des Dichlormethans am Rotationsverdampfer wurde das überschüssige Silylchlorid destillativ entfernt (50 °C, 10⁻³ mbar), wobei das Produkt als farbloser Feststoff ausfiel.
Ausbeute: 98% (11,5 g, 29,6 mmol)
GC (Methode *hart,* HP-5): t*_{R}* = 14.5 min
R*_{f}* (CH:EE = 5:1): 0,68
HRMS (ESI, pos.mode): *m*/*z* für CHO [M+Na⁺]:
Berechnet: 411,1968
Gefunden: 411,1960
Schmelzpunkt: 67,8 °C
¹H-NMR (400 MHz, CDCl₃): δ [ppm] = -0.12 (s, 6H, 9, 10-H), 0.69 (s, 9H, 11-H, 12-H, 13-H), 2.30 (s, 3H, 8-H), 3.73 (s, 3H, 8'-H), 3.80 (s, 3H, 7'-H), 3.83 (s, 3H, 7-H), 6.50-6.53 (m, 2H, 3'-H, 5'-H), 6.65-6.68 (m, 1H, 4-H), 7.11-7.14 (m, 2H, 6-H, 6'-H)
¹³C-NMR (101 MHz, CDCl₃): δ [ppm] = -4.51 (C-9,C-10), 18.47 (C-14), 21.35 (C-8), 25.68 (C-11, C-12, C-13), 55.12 (C-8'), 55.55 (C-7'), 55.62 (C-7), 98.70 (C-3'), 103.95 (C-5'), 111.59 (C-4), 121.38 (C-1'), 124.12 (C-6), 129.91 (C-6'), 130.51 (C-1), 132.39 (C-5), 140.53 (C-2), 150.41 (C-3), 158.18 (C-4'), 160.31 (C-2')

### Beispiel 6:

### Derivatisierung der Phenol-Aren-Komponenten aus Beispiel 2 durch Schutzgruppen Schützung mit Tri(methylethyl)-silylchlorid (TIPS-chlorid)

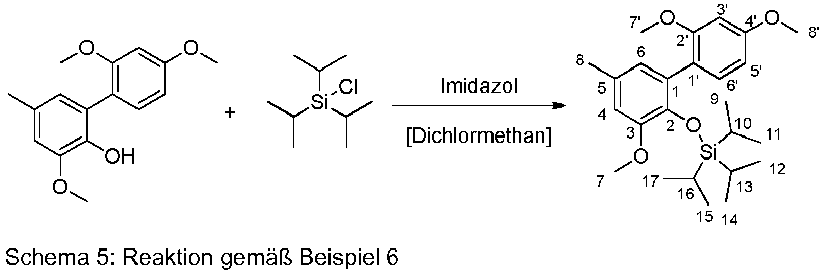

In einem Rundkolben mit Trockenrohr wurden 5,27 g Imidazol (27,3 mmol, 2,5 Äqu.) vorgelegt. 8,50 g (31,0 mmol, 1,0 Äqu.) der AB-Verbindung aus Beispiel 2 (2-Hydroxy-5-methyl-2',3,4'-trimethoxybiphenyl wurden in ca. 60 mL trockenem Dichlormethan gelöst und in den Kolben überführt. Anschließend wurden 11,57 g (62,0 mmol, 2,0 Äqu.) TIPS-Chlorid ebenfalls in den Ansatz hinzugegeben, wobei eine orange, gelbliche Lösung entstand. Der Ansatz wurde für 24 h bei RT gerührt. Anschließend wurden weitere 0.5 Äqu. TIPS-Chlorid hinzugegeben und der Ansatz für weitere 48 h bei Raumtemperatur gerührt. Während der Reaktionszeit entstand ein farbloser, flockiger Niederschlag. Die Isolierung des Produktes erfolgte mittels dreimaliger Extraktion mit je 100 mL Wasser, um das entstandene Salz abzutrennen. Nach Trocknung mittels Natriumsulfat und Entfernung des Dichlormethans am Rotationsverdampfer wurde das überschüssige Silylchlorid destillativ entfernt (50 °C, 10⁻³ mbar), wobei das Produkt als farbloser Feststoff ausfiel.
Ausbeute: 92% (13,3 g, 30,9 mmol)
GC (Methode *hart,* HP-5): t*_{R}* = 15,8 min
R*_{f}* (CH:EE = 5:1): 0,73
HRMS (ESI, pos.mode): *m*/*z* für CHO [M+Na⁺]:
Berechnet: 453,2437
Gefunden: 453,2423
Schmelzpunkt: 78,4 °C
¹H-NMR (400 MHz, CDCl₃): δ [ppm] = 0.87 (d, 18 H, 9-H, 11-H, 12-H, 14-H, 15-H, 17-H), 1.56 (q, 3H, 10-H, 13-H, 16-H), 2.29 (s, 3H, 8-H), 3.70 (s, 3H, 8'-H), 3.78 (s, 3H,7'-H), 3.83 (s, 3H, 7-H), 6.59-6.61 (m, 2H, 3'-H, 5'-H), 6.62-6.65 (m, 1H, 4-H), 7.09-7.12 (m, 2H, 6-H, 6'-H)
¹³C-NMR (101 MHz, CDCl₃): δ [ppm] = 13.69 (C-8), 17.73 (C-9, C-11, C-12, C-14, C-15, C-17), 21.00 (C-10, C-13, C-16), 54.47 (C-8'), 55.08 (C-7'), 55.32 (C-7), 98.23 (C-3'), 103.48 (C-5'), 110.87 (C-4), 121.39 (C-1'), 123.61 (C-6), 128.81 (C-6'), 129.70 (C-1), 131.53 (C-5), 141.02 (C-2), 149.23 (C-3), 157.86 (C-4'), 159.93 (C-2')

### Beispiel 7

### Darstellung von 2,2"-Dihydroxy-3"-(1,1-Dimethylethyl) -5-methyl-2',3,4',5"-tetramethoxy-[1,1',5',1"]-terphenyl

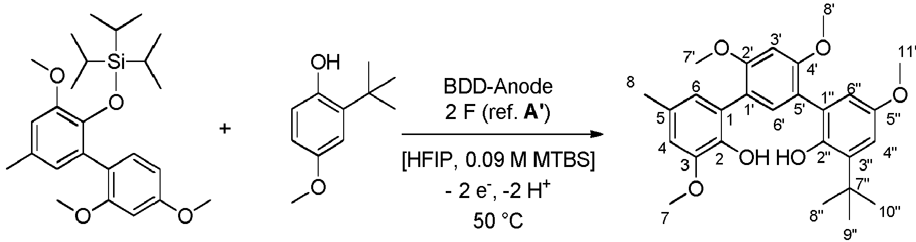

Die Elektrolyse wurde gemäß der allgemeinen Vorschrift *AAV1* in einer Flansch-Zelle durchgeführt. Dafür wurde ein Elektrolytvolumen von 33 mL HFIP mit 1,03 g MTBS als Leitsalz verwendet (0,09 M). Zur elektrochemischen Kreuzkupplung wurden 0,90 g (5,0 mmol, 1.0 Äqu.) 2-(1,1-Dimethylethyl)-4-methoxyphenol und 6,45 g (15 mmol, 3,0 Äqu.) des geschützten Phenol-Aren-Kupplungsproduktes aus Beispiel 6 eingesetzt. Die Elektrolyse wurde galvanostatisch durchgeführt, wobei eine tiefgefärbte Lösung entstand. Nach beendeter Elektrolyse wurde das Lösemittel unter vermindertem Druck am Rotationsverdampfer entfernt und der braune Rückstand mit ca. 300 mL Ethylacetat über Kieselgel 60 filtriert. Die abschließende säulenchromatographische Auftrennung (Säulenabmessung: 50x3 cm) des Produktgemisches über Kieselgel 60 als Flashchromatographie wurde zweimal mit folgendem Gradienten im Laufmittelgemisch durchgeführt: 95:5 zu 9:1 zu 5:1 zu 2:1 (CH:EE, ca. 3 L CH und 2 L EE je Säule verwendet). Dabei wurde das entsprechende Produkt mit entfernter Schutzgruppe als tiefroter Feststoff erhalten.
Ausbeute: 0,91 g an ungeschützten Pincerliganden, (40%, 2,01 mmol)
GC (Methode *hart,* HP-5): t*_{R}* = 22,34 min
R*_{f}* (CH:EE = 5:1): 0,10
HRMS (ESI, pos.mode): *m*/*z* für CHO [M+Na⁺]:
Berechnet: 475,2097
Gefunden: 475,2103
¹H-NMR (400 MHz, CDCl₃): δ [ppm] = 1.44 (s, 9H, 8"-H, 9"-H, 10"-H), 2.31 (s, 3H, 8-H), 3.75 (s, 3H, 11"-H), 3.91 (s, 3H, 8'-H), 3.91 (s, 3H, 7'-H) 3.93 (s, 3H, 7-H), 5.79 (s, 1 H, 2"-H), 5.89 (s, 1 H, 2-H), 6.65 (d, 1 H, 3'-H), 6.67-6.72 (m, 3H, 4-H, 6-H, 6"-H), 6.91 (d, 1 H, 4"-H), 7.30 (s, 1 H, 6'-H)
¹³C-NMR (101 MHz, CDCl₃): δ [ppm] = 20.90 (C-8), 29.51 (C-8", C-9", C-10"), 34.98 (C-7"), 55.50 (C-11 "), 55.86 (C-8'), 56.16 (C-7'), 56.32 (C-7), 96.27 (C-3'), 111.04 (C-6"), 112.42 (C-4"), 113.59 (C-4), 119.79 (C-5'), 120.65 (C-1'), 123.49 (C-6), 124.20 (C-1), 126.59 (C-1"), 129.02 (C-6'), 135.15 (C-5), 138.61 (C-3"), 140.72 (C-2"), 146.22 (C-3), 146.84 (C-2), 152.55 (C-5"), 156.03 (C-2'), 157.13 (C-4')

### Beispiel 8:

### Darstellung von 2, 2"-Dihydroxy-4",5-dimethyl-2',3,4',5"-tetramethoxy- [1,1',5',1"]-terphenyl

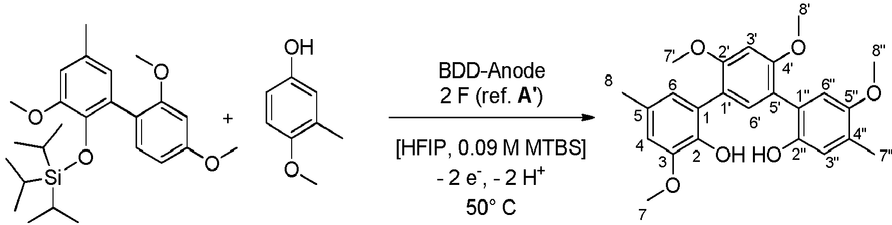

Die Elektrolyse wurde gemäß der allgemeinen Vorschrift *AAV1* in einer Flansch-Zelle durchgeführt. Dafür wurde ein Elektrolytvolumen von 33 mL HFIP mit 1,03 g MTBS als Leitsalz verwendet (0,09 M). Zur elektrochemischen Kreuzkupplung wurden 0,69 g (5,0 mmol, 1,0 Äqu.) des 4-Methoxy-3-methylphenols und 6,45 g (15 mmol, 3,0 Äqu.) des geschützten Phenol-Aren-Kupplungsprodukts aus Beispiel 6 eingesetzt. Die Elektrolyse erfolgte galvanostatisch, wobei eine schwarze Lösung entstand. Nach beendeter Elektrolyse wurde das Lösemittel unter vermindertem Druck am Rotationsverdampfer entfernt und die zurückbleibende braune Lösung mit ca. 300 mL Ethylacetat über Kieselgel 60 filtriert. Die abschließende säulenchromatographische Auftrennung (Säulenabmessung: 50x3 cm) des Produktgemisches über Kieselgel 60 als Flashchromatographie wurde zweimal mit folgendem Gradienten im Laufmittelgemisch durchgeführt: 95:5 zu 9:1 zu 5:1 zu 2:1 (CH:EE, ca. 3 L CH und 2 L EE je Säule verwendet). Dabei wurde das entsprechende Produkt mit entfernter Schutzgruppe als tiefroter Feststoff erhalten.
Ausbeute: 0,88 g an ungeschützten Pincerliganden, (43%, 2,15 mmol)
GC (Methode *hart,* HP-5): t*_{R}* = 20,3 min
R*_{f}* (CH:EE = 5:1): 0,1
HRMS (ESI, pos.mode): *m*/*z* für CHO [M+H⁺]:
Berechnet: 411,1802
Gefunden: 411,1797
¹H-NMR (400 MHz, CDCl₃): δ [ppm] = 2.22 (s, 3H, 7'-H'), 2.31 (s, 3H, 8-H), 3.76 (s, 3H, 8"-H), 3.91 (s, 3H, 8'-H), 3.91 (s, 3H, 7'-H), 3.96 (s, 3H, 7-H), 5.79 (s, 1 H, 2"-H), 5.93 (bs, 1 H, 2-H), 6.67-6.74 (m, 3'-H, 4-H, 6-H, 6"-H), 6.83 (s, 1 H, 3"-H), 7.31 (s, 1 H, 6'-H)
¹³C-NMR (101 MHz, CDCl3): δ [ppm] = 15.79 (C-7"), 20.99 (C-8), 55.83 (C-8"), 55.85 (C-8'), 56.15 (C-2'), 56.44 (C-7), 96.26 (C-3'), 110.99 (C-4), 112.62 (C-6"), 119.62 (C-3"), 120.69 (C-4"), 122.17 (C-5'), 123.37 (C-1'), 124.15 (C-6), 127.43 (C-1"), 129.03 (C-1), 134.79 (C-6'), 140.77 (C-5), 146.84 (C-2"), 151.88 (C-5"), 155.75 (C-3), 157.05 (C-2), 158.29 (C-4'), 158.43 (C-2')

Wie die Beispiele 7 und 8 zeigen, konnte die Ausbeute an m-Terphenylverbindung durch das Einführen einer Schutzgruppe (Silygruppe) in das Phenol-Aren-Kupplungsprodukt bei der anschließenden Kupplung im elektrochemisch durchgeführten Verfahrensschritt c) weiter erhöht werden (doppelt bis dreifach erhöhte Ausbeute). Auf diese Weise kann somit die Effizienz des Gesamtverfahrens deutlich verbessert werden.

## Patentansprüche

1. Verfahren zur Herstellung unsymmetrischer Verbindungen aus der Gruppe der m-Terphenyle umfassend die Verfahrensschritte
a) Umsetzen eines ersten substituierten oder unsubstituierten Phenols mit einem 1,3-disubstituierten Aren, welches in 2 und 5 Stellung ebenfalls substituiert sein kann, unter Erhalt eines Phenol-Aren-Kupplungsprodukts und
b) optional Schützen der OH-Gruppe des Phenol-Aren-Kupplungsprodukts mit einer Schutzgruppe unter Erhalt eines geschützten Phenol-Aren-Kupplungsprodukts
c) Umsetzen des Phenol-Aren-Kupplungsprodukts aus a) oder b) mit einem zweiten substituierten oder unsubstituierten Phenol unter Erhalt eines unsymmetrischen m-Terphenyls,
mit der Maßgabe, dass das erste Phenol und das zweite Phenol unterschiedlich substituiert sind,
**dadurch gekennzeichnet,**
**dass** mindestens einer der Verfahrensschritte a) oder c) als elektrochemischer Verfahrensschritt durchgeführt wird.

2. Verfahren nach Anspruch 1,
**dadurch gekennzeichnet,**
**dass** beide Verfahrensschritte a) und c) als elektrochemische Verfahrensschritte durchgeführt werden.

3. Verfahren nach Anspruch 1 oder 2,
**dadurch gekennzeichnet,**
**dass** der elektrochemische Verfahrensschritt so durchgeführt wird, dass
aa) eine Mischung mindestens eines Lösemittels und mindestens eines Leitsalzes erzeugt wird,
bb) zu dieser Mischung die umzusetzenden Verbindungen gegeben werden, wobei das Phenol im molaren Unterschuss bezogen auf die andere umzusetzende Komponente zugegeben wird,
cc) Einbringen von mindestens zwei Elektroden in die unter bb) erhaltene Reaktionslösung und Anlegen einer Spannung an die Elektroden,
dd) Umsetzen der Komponenten zu den entsprechenden Kupplungsprodukten,
ee) Abschalten der Spannung, und ggf.
ff) Isolierung und/oder Aufreinigung des Umsetzungsproduktes.

4. Verfahren nach einem der Ansprüche 1 bis 3,
**dadurch gekennzeichnet,**
**dass** das molare Verhältnis des 1,3-disubstituierten Arens, welches in 2 und 5 Stellung ebenfalls substituiert sein kann, zum ersten substituierten oder unsubstituierten Phenol im Bereich von 1,5 : 1 bis 6 : 1, bevorzugt von 2 : 1 bis 3 : 1 liegt.

5. Verfahren nach einem der Ansprüche 1 bis 4,
**dadurch gekennzeichnet,**
**dass** das molare Verhältnis des Phenol-Aren Kupplungsproduktes zum zweiten Phenol im Bereich von 1,5 : 1 bis 6 : 1, bevorzugt von 2 : 1 bis 3 : 1 liegt.

6. Verfahren nach einem der Ansprüche 1 bis 5,
**dadurch gekennzeichnet,**
**dass** das erste substituierte oder unsubstituierte Phenol der Formel (A) 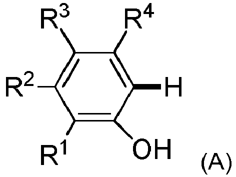 das zweite substituierte oder unsubstituierte Phenol der Formel (A') 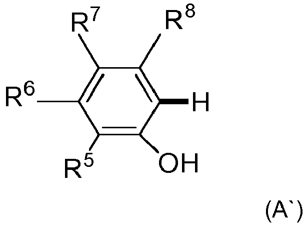 und das 1,3-disubstituierte Aren, welches in 2 und 5 Stellung ebenfalls substituiert sein kann, der Formel (B) 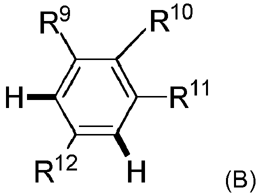 entspricht,
und R¹, R², R³, R⁴, R⁵, R⁶, R⁷, R⁸, R¹⁰ und R¹² unabhängig voneinander ausgewählt sind aus der Gruppe bestehend aus Wasserstoff, (C₁-C₁₂)-Alkyl, O-(C₁-C₁₂)-Alkyl, (C₆-C₂₀)-Aryl, O-(C₆-C₂₀)-Aryl und Halogen, vorzugsweise bestehend aus Wasserstoff, (C₁-C₁₂)-Alkyl, O-(C₁-C₁₂)-Alkyl und Halogen, besonders bevorzugt bestehend aus Wasserstoff, (C₁-C₁₂)-Alkyl, O-(C₁-C₁₂)-Alkyl,
worin R⁹ und R¹¹ unabhängig voneinander ausgewählt sind aus der Gruppe bestehende aus (C₁-C₁₂)-Alkyl, O-(C₁-C₁₂)-Alkyl, (C₆-C₂₀)-Aryl, O-(C₆-C₂₀)-Aryl und, Halogen, vorzugsweise bestehend aus (C₁-C₁₂)-Alkyl, O-(C₁-C₁₂)-Alkyl und Halogen, besonders bevorzugt bestehend aus (C₁-C₁₂)-Alkyl, O-(C₁-C₁₂)-Alkyl, und worin innerhalb der Paare R¹/ R⁵, R²/R⁶, R³/R⁷ oder R⁴/R⁸ mindestens ein Paar aus verschiedenen Resten besteht.

7. Verfahren nach Anspruch 6,
wobei R¹⁰ und R¹² identisch sind und vorzugsweise Wasserstoff bedeuten.

8. Verfahren nach einem der Ansprüche 1 bis 7,
**dadurch gekennzeichnet,**
**dass** der elektrochemische Verfahrensschritt in Gegenwart eines Lösemittels durchgeführt wird und als Lösemittel ein Lösemittel aus der Gruppe von Acetonitril, Propylencarbonat, Methylcarbonat, Nitromethan, Ethylenglycoldimethylether, Methansulfonsäure, Benzol, Toluol, Wasser, Methanol, Ethanol, Propanol, Isopropanol, halogenierte Lösemittel oder halogenierte oder nicht halogenierte Säuren oder Mischungen dieser eingesetzt wird.

9. Verfahren nach Anspruch 8,
**dadurch gekennzeichnet,**
**dass** als Lösemittel Methanol, Ameisensäure Trifluoressigsäure, Hexafluorisopropanol oder Mischungen dieser, vorzugsweise Methanol, Hexafluorisopropanol oder Mischungen dieser, und bevorzugt Hexafluorisopropanol verwendet wird.

10. Verfahren nach einem der Ansprüche 1 bis 9,
**dadurch gekennzeichnet,**
**dass** die Umsetzung ohne die Verwendung von organischen Oxidationsmitteln durchgeführt wird.

11. Verfahren nach einem der Ansprüche 1 bis 10,
**dadurch gekennzeichnet,**
**dass** der elektrochemische Verfahrensschritt in Gegenwart mindestens eines Leitsalzes durchgeführt wird, wobei als Leitsalze solche eingesetzte werden, die ausgewählt sind aus der Gruppe von Tetra(C₁-C₆-alkyl)-ammonium- oder (1,3-Di(C₁-C₆-alkyl)imidazoliumsalzen, mit der Maßgabe, dass die alkyl-Gruppen Halogen-substituiert sein können.

12. Verfahren nach Anspruch 11,
**dadurch gekennzeichnet,**
**dass** die Gegenionen der Leitsalze ausgewählt sind aus der Gruppe umfassend Arsenat, Sulfat, Hydrogensulfat, Alkylsulfat, Alkylphosphat, Perchlorat, Fluorid, Arylsulfat, Hexafluorphosphat und Tetrafluorborat.

13. Verfahren nach einem der Ansprüche 1 bis 12,
wobei als Leitsalz ein Salz aus der Gruppe umfassend quaternäre Ammoniumborate, Ammoniumfluoralkylphosphate, Ammoniumfluoralkylarsenate, Ammoniumtrifluor-methylsulfonate, Ammoniumbis(fluoromethansulfon)imide, Ammoniumtris- (fluoromethansulfonyl)methide, Methyltributylammoniummethylsulfat, Methyltriethylammoniummethylsulfat, Tetrabutyl-ammoniumhexafluorophosphat, Tetraethylammoniumtetrafluoroborat, Lithiumhexafluorophosphat oder Tetraethylammoniumtetrafluoroborat, bevorzugt Methyltriethylammoniummethylsulfat oder Bu3NMe+MeOSO3-(Methyltributylammoniummethylsulfat) und besonders bevorzugt Methyltributylammoniummethylsulfat eingesetzt wird.

14. Verbindungen der Formel (CDC') 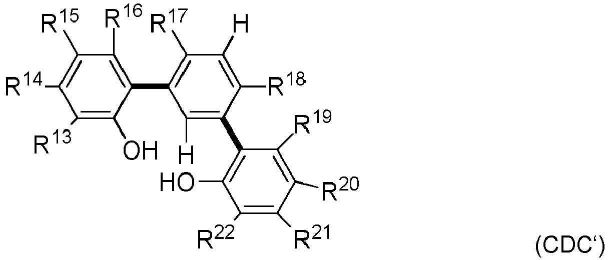
worin die Reste R¹³, R¹⁴, R¹⁵, R¹⁶, R¹⁹, R²⁰, R²¹ und R²² unabhängig voneinander ausgewählt sind aus der Gruppe bestehende aus Wasserstoff, (C₁-C₁₂)-Alkyl, O-(C₁-C₁₂)-Alkyl, (C₆-C₂₀)-Aryl, O-(C₆-C₂₀)-Aryl, Halogen, vorzugsweise bestehend aus Wasserstoff, (C₁-C₁₂)-Alkyl, O-(C₁-C₁₂)-Alkyl, Halogen, besonders bevorzugt bestehend aus Wasserstoff, (C₁-C₁₂)-Alkyl und O-(C₁-C₁₂)-Alkyl,
worin R¹⁷ und R¹⁸ unabhängig voneinander ausgewählt sind aus der Gruppe bestehende aus (C₁-C₁₂)-Alkyl, O-(C₁-C₁₂)-Alkyl, (C₆-C₂₀)-Aryl, O-(C₆-C₂₀)-Aryl und Halogen, vorzugsweise bestehend aus (C₁-C₁₂)-Alkyl, O-(C₁-C₁₂)-Alkyl und Halogen, besonders bevorzugt bestehend aus (C₁-C₁₂)-Alkyl und O-(C₁-C₁₂)-Alkyl, und
worin innerhalb der Paare R¹³/R²², R¹⁴/R²¹, R¹⁵/R²⁰ oder R¹⁶/R¹⁹ mindestens ein Paar aus verschiedenen Resten besteht.

15. Verbindungen gemäß Anspruch 14,
**dadurch gekennzeichnet,**
**dass** sie den Formeln (1), (2) oder (3) 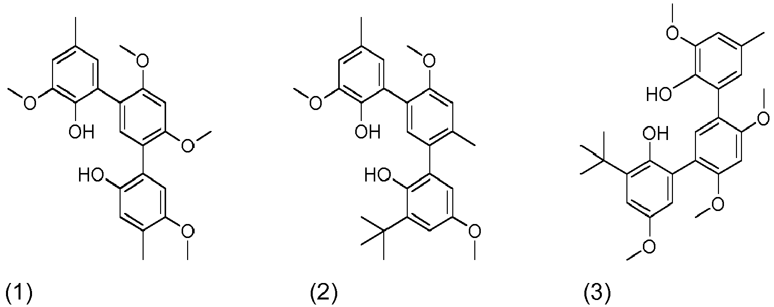 genügen.

16. Verwendung einer Verbindung gemäß Anspruch 14 oder 15 als Ligand zur Herstellung von Metall-Ligand-Katalysatorsystemen.
